# EUROPEAN PATENT APPLICATION

(11) **EP 3 168 291 A1**
(43) Date of publication of application: **17.05.2017**
(21) Application number: 15819252.6
(22) Date of filing: 10.07.2015
(51) Int. Cl.: C12M 3/00, C12M 1/00

(54) **CELL CULTURE SYSTEM**

(30) Priority: 10.07.2014 JP 2014142253; 10.10.2014 JP 2014208776; 28.10.2014 JP 2014218777
(71) Applicant: OLYMPUS CORPORATION, Hachioji-shi Tokyo 192-8507 (JP)
(72) Inventor: KIMURA, Hiroyuki, Tokyo 192-8507 (JP); MINAMI, Tatsuya, Tokyo 192-8507 (JP); MAKARA, Yasunori, Tokyo 192-8507 (JP)
(74) Representative: Schicker, Silvia
(86) International application number: PCT/JP2015/069869
(87) International publication number: WO 2016/006680

(57) **Abstract**

Provided is a cell-culturing system provided with: a solution-holding means that holds a medium for culturing cells; and a solution-supplying means that is connected to the solution-holding means and that supplies the solution supplied thereto from the solution-holding means to a cell-culturing container. The solution-supplying means may supply a solution to the cell-culturing container via a solution dripping step. The cell-culturing container may be connected to the solution-supplying means at a position that is lower than the solution-supplying means in a direction in which gravity acts; the solution-supplying means may have a space in which the solution supplied thereto from the solution-holding means is made to drip; a cell-culturing device may be provided with a discharging port that discharges the solution at a position in a container side surface at a desired height; and the solution may be discharged outside when the height of a top surface of the solution in the cell-culturing container reaches the discharging port as a result of supplying the solution to the cell-culturing container from the solution-supplying means.

## Description

### {Technical Field}

The present invention relates to a cell-culturing system in which a medium in a cell-culturing container is replaced.

### {Background Art}

In recent years, with the progress made in stem cell research and regenerative medicine, there has been a demand for preparing a large amount of cells for clinical use. Work involved in preparing cells for clinical use has been required to be performed in an environment that meets strict standards, and, because of this, considerable time and effort, as well as costs, are incurred; for example, an operator needs to change into disposable work clothes when he/she enters a workspace. In addition, the work procedures performed by the operator create situations in which a culturing system may become polluted. Therefore, there is a demand for reducing, as much as possible, the number of times that the operator enters and works in the workspace and for automatically performing work when possible to reduce the dependence on manual work.

Although a medium (cell-culturing liquid) needs to be periodically replaced in order to culture cells, medium replacement is associated with a risk of polluting (contaminating) the culturing system, and thus, it is desirable that the work be automatically performed without manual intervention as much as possible. As a system in which a medium is automatically replaced, there is a known system in which the medium is continuously refluxed in the culturing system (Patent Literature 1).

### {Citation List}

### {Patent Literature}

{PTL 1} Japanese Unexamined Patent Application, Publication No. Sho 61-58582

### {Summary of Invention}

### {Technical Problem}

Strict standards are set for preparing cells for clinical use, and, although it is necessary to eliminate the risk of polluting the culturing system as much as possible, with a system in which the medium is continuously refluxed, such as the one in Patent Literature 1, there is a risk of polluting the culturing system due to a reverse flow of deteriorated medium caused by problems in refluxing or the like.

The present invention has been conceived in light of the above-described circumstances, and an object thereof is to provide a cell-culturing system with which it is possible to replace, in a simple manner, a medium in a cell-culturing container in a cell-culturing space, and with which it is also possible to reduce the risk of polluting the cell-culturing system.

### {Solution to Problem}

In order to achieve the above-described object, the present invention provides the following solutions. A first aspect of the present invention provides a cell-culturing system including: a solution-holding means that holds a medium for culturing cells; and a medium-supplying means that is connected to the solution-holding means and that supplies the medium supplied thereto from the solution-holding means to a cell-culturing container, wherein the cell-culturing container is connected to the medium-supplying means at a position that is lower than the medium-supplying means in a direction in which gravity acts, the medium-supplying means has a space in which the medium supplied thereto from the solution-holding means is made to drip, the cell-culturing container is provided with a discharging port that discharges the medium at a position in a container side surface at a desired height, and the discharging port discharges the medium outside when the height of a top surface of the medium reaches the discharging port as a result of supplying the medium from the medium-supplying means.

With this aspect, it is possible to supply/discharge the solution such as a medium or the like to/from the cell-culturing container even when the user is not on site. In addition, because the medium is made to drip in the dripping container, it is possible to reduce the risk of polluting the medium in the solution-holding means upstream therefrom.

A second aspect of the present invention provides a cell-culturing system including: a solution-holding means that holds a medium for culturing cells; and a medium-supplying means that is connected to the solution-holding means and that supplies the medium supplied thereto from the solution-holding means to a cell-culturing container, wherein the cell-culturing container is provided with a supply port to which the medium is supplied from the medium-supplying means at a container top surface, and is also provided with a discharging port that discharges the medium at a position in a container side surface at a desired height, the medium supplied from the supply port is made to drip from the supply port in a space in the cell-culturing container toward a top surface of the medium, and the discharging port discharges the medium outside when the height of the top surface of the medium reaches the discharging port as a result of supplying the medium from the medium-supplying means.

With this aspect, it is possible to supply/discharge the solution such as a medium or the like to/from the cell-culturing container even when the user is not on site. In addition, because the medium is made to drip in the cell-culturing container, it is possible to reduce the risk of polluting the medium in the solution-holding means and the medium-supplying means upstream therefrom.

In addition, the individual aspects described above may be provided with a dripping-speed adjusting means that controls the dripping speed of the solution. By doing so, it is possible to replace an old medium in the cell-culturing container at arbitrary replacement efficiency, and thus, it is possible to slow down the speed at which the medium is deteriorated.

In addition, the aspects provided with the above-described dripping-speed adjusting means may be provided with a control portion that remotely controls the dripping-speed adjusting means. By doing so, it is possible to reduce the number of times that an operator enters and works in a workspace, and thus, it is possible to reduce the time and effort, as well as costs, associated with the work and also to decrease the chance of the culturing system becoming polluted.

In addition, the individual aspects described above may be provided with a pressurizing means that applies pressure to the medium in the medium-preserving means. By doing so, the adjusting range of the dripping speed is increased, and thus, it is possible to more precisely control the dripping speed. In addition, the degree of freedom with respect to the positions at which the medium-preserving means and the temporary medium-holding means are installed is increased.

In addition, the individual aspects described above may be provided with a negative-pressure supplying means that applies negative pressure to the discharging port of the cell-culturing container. By doing so, the degree of freedom with respect to the positions at which the discharging port of the cell-culturing container is installed is increased, and thus, it is possible to increase the forms of cell-culturing containers that can be used.

In addition, the individual aspects described above may additionally be provided with a waste-liquid holding means that is connected to the discharging port of the cell-culturing container and that holds the medium supplied from the cell-culturing container, and the medium discharged from the cell-culturing container may be made to drip in a space in the waste-liquid holding means. By doing so, because the waste liquid is made to drip in the waste-liquid holding means, it is possible to prevent reverse flow thereof, and thus, it is possible to reduce the risk of polluting the cell-culturing container.

A third aspect of the present invention provides a cell-culturing system including: a solution-holding means that holds a solution, such as a medium or the like for culturing cells; a solution-supplying means that is connected to the solution-holding means and that supplies the solution supplied thereto from the solution-holding means to a cell-culturing container; and a solution-discharging means that discharges the solution from the cell-culturing container, wherein the cell-culturing container is connected to the solution-supplying means at a position that is lower than the solution-supplying means in a direction in which gravity acts, and the solution-supplying means has a space in which the solution supplied thereto from the solution-holding means is made to drip.

With this aspect, because the solution is made to drip in the solution-supplying means, it is possible to reduce the risk of polluting the solution, such as a medium or the like, in the solution-holding means upstream therefrom.

A fourth aspect of the present invention provides a cell-culturing system including: a solution-holding means that holds a solution, such as a medium or the like for culturing cells; a solution-supplying means that is connected to the solution-holding means and that supplies the solution supplied thereto from the solution-holding means to a cell-culturing container; and a solution-discharging means that discharges the solution from the cell-culturing container, wherein the cell-culturing container is connected to the solution-supplying means at a position that is lower than the solution-supplying means in a direction in which gravity acts, and a supply port to which the solution is supplied from the solution-supplying means is provided at a top surface of the cell-culturing container, and the solution supplied from the supply port is made to drip from the supply port in the space in the cell-culturing container.

With this aspect, because the solution is made to drip in the cell-culturing container, it is possible to reduce the risk of polluting the solution, such as a medium or the like, in the solution-holding means upstream therefrom.

In addition, in the above-described third and fourth aspects, the solution-supplying means may be provided with a dripping-speed adjusting means that controls a dripping speed of the solution; the solution-discharging means may be provided with a waste-liquid-speed adjusting means that controls a discharging speed of the solution; and a control portion that remotely performs the control of the dripping speed of the solution by means of the dripping-speed adjusting means and the control of the discharging speed of the solution by means of the waste-liquid-speed adjusting means.

By doing so, because the operator can remotely replace the medium in the culturing system, it is possible to reduce the number of times that the operator enters and works in the workspace, and thus, it is possible to reduce the time and effort, as well as costs, associated with the work and also to decrease the chance of the culturing system becoming polluted.

In addition, the above-described third and fourth aspects may be provided with a pressurizing means that applies pressure to the solution in the solution-holding means. By doing so, the adjusting range of the dripping speed is increased, and thus, it is possible to more precisely control the dripping speed. In addition, the degree of freedom with respect to the positions at which the solution-preserving means is installed is increased.

In addition, in the above-described third and fourth aspects, the cell-culturing container may be provided with a discharging port that is connected to the solution-discharging means, and a negative-pressure supplying means that applies negative pressure to the discharging port of the cell-culturing container may be provided. By doing so, the adjusting range of the discharging speed of the solution from the cell-culturing container is increased, and thus, it is possible to more precisely control the discharging speed. In addition, the degree of freedom with respect to the positions at which the discharging port of the cell-culturing container is installed is increased, and thus, it is possible to increase the forms of cell-culturing containers that can be used.

In addition, in the above-described third and fourth aspects, the solution-discharging means may be provided with a waste-liquid holding means that is connected to the cell-culturing container and that holds the solution discharged from the cell-culturing container; and the solution discharged from the cell-culturing container may be made to drip in the space in the waste-liquid holding means. By doing so, because the waste liquid is made to drip in the waste-liquid holding means, it is possible to prevent reverse flow thereof, and thus, it is possible to reduce the risk of polluting the cell-culturing container.

Another aspect of the present invention provides a cell-culturing system including: a solution-holding means that holds a solution, such as a medium or the like for culturing cells; and a solution-supplying means that is connected to the solution-holding means and that supplies the solution supplied thereto from the solution-holding means to a cell-culturing container wherein the solution-supplying means supplies the solution to the cell-culturing container via a solution dripping step.

With this aspect, because the solution, such as a medium or the like, is made to drip in the cell-culturing container, it is possible to reduce the risk of polluting the medium in the solution-holding means upstream therefrom.

Another aspect of the present invention provides a cell-culturing system including: a solution-holding means that holds a solution, such as a medium or the like for culturing cells; a solution-supplying means that is connected to the solution-holding means and that supplies the solution supplied thereto from the solution-holding means to a cell-culturing container; and a solution-discharging means that discharges the solution from the cell-culturing container, wherein the solution-discharging means discharges the solution from the cell-culturing container via a solution dripping step.

With this aspect, it is possible to supply/discharge the solution such as a medium or the like to/from the cell-culturing container even when the user is not on site.

Another aspect of the present invention provides a cell-culturing system including: a medium-preserving means that maintains a medium for culturing cells at an appropriate temperature for preserving the medium; a temporary medium-holding means that is connected to the medium-preserving means and that maintains the medium supplied thereto from the medium-preserving means at an appropriate temperature for cell culturing; a medium-supplying means that is connected to the temporary medium-holding means and that supplies the medium supplied thereto from the temporary medium-holding means to a cell-culturing container; and a cell-culturing container that is connected to the medium-supplying means at a position that is lower than the medium-supplying means in a direction in which gravity acts and in which cells are cultured in the medium supplied thereto from the medium-supplying means, wherein the medium-supplying means is provided with a dripping container having space in which the medium supplied thereto from the temporary medium-holding means is made to drip, and supplies the medium that is made to drip in the dripping container to the cell-culturing container, and the cell-culturing container is provided with a discharging port that discharges the medium at a position in a container side surface at a desired height; and the discharging port discharges the medium outside when the height of a top surface of the medium reaches the discharging port as a result of supplying the medium from the medium-supplying means.

With this aspect, it is possible to automatically supply/discharge the solution such as a medium or the like to/from the cell-culturing container even when the user is not on site. In addition, because the medium is made to drip in the dripping container, it is possible to reduce the risk of polluting the medium in the medium-preserving means and the temporary medium-holding means upstream therefrom.

Another aspect of the present invention provides a cell-culturing system including: a medium-preserving means that maintains a medium for culturing cells at an appropriate temperature for preserving the medium; a temporary medium-holding means that is connected to the medium-preserving means and that maintains the medium supplied thereto from the medium-preserving means at an appropriate temperature for cell culturing; a medium-supplying means that is connected to the temporary medium-holding means and that supplies the medium supplied thereto from the temporary medium-holding means to a cell-culturing container; and a cell-culturing container that is connected to the medium-supplying means at a position that is lower than the medium-supplying means in a direction in which gravity acts and in which cells are cultured in the medium supplied thereto from the medium-supplying means, wherein the cell-culturing container is provided with a supply port to which the medium is supplied from the medium-supplying means at the container top surface and a discharging port that discharges the medium at a position in a container side surface at a desired height; the medium supplied from the supply port is made to drip from the supply port in the space in the cell-culturing container toward a top surface of the medium; and the discharging port discharges the medium outside when the height of the top surface of the medium reaches the discharging port as a result of supplying the medium from the medium-supplying means.

With this aspect, it is possible to automatically supply/discharge the solution such as a medium or the like to/from the cell-culturing container even when the user is not on site. In addition, because the medium is made to drip in the cell-culturing container, it is possible to reduce the risk of polluting the medium in the medium-preserving means, the temporary medium-holding means, and the medium-supplying means upstream therefrom.

### {Advantageous Effects of Invention}

With the present invention, it is possible to remotely replace a medium in a cell-culturing system even if an operator is not on site, and thus, it is possible to reduce the number of times that the operator enters a workspace. By doing so, it is possible to reduce the time and effort, as well as costs, involved in changing into disposable work clothes or the like, and it is also possible to reduce the risk of contaminating the cell-culturing system with bacteria or the like.

### {Brief Description of Drawings}

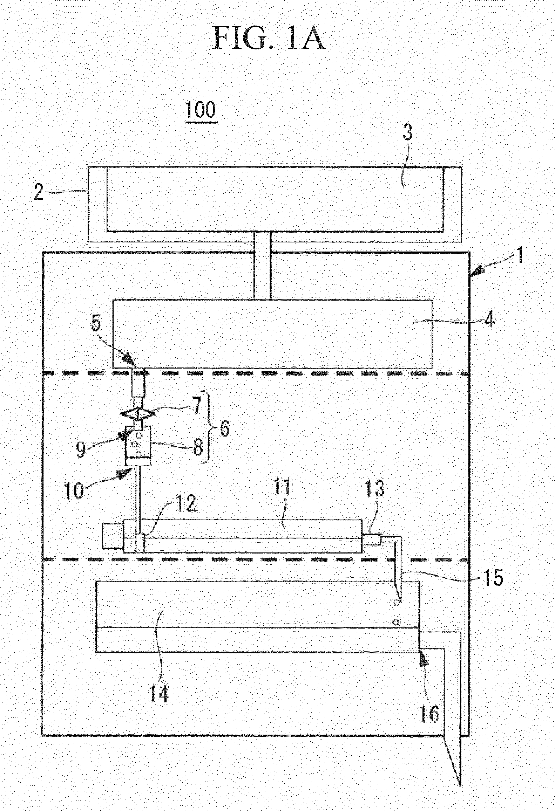

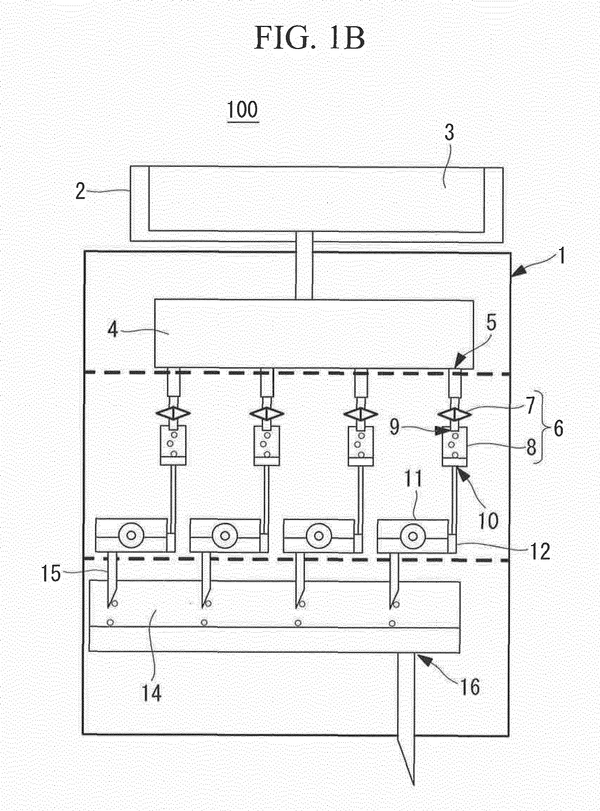

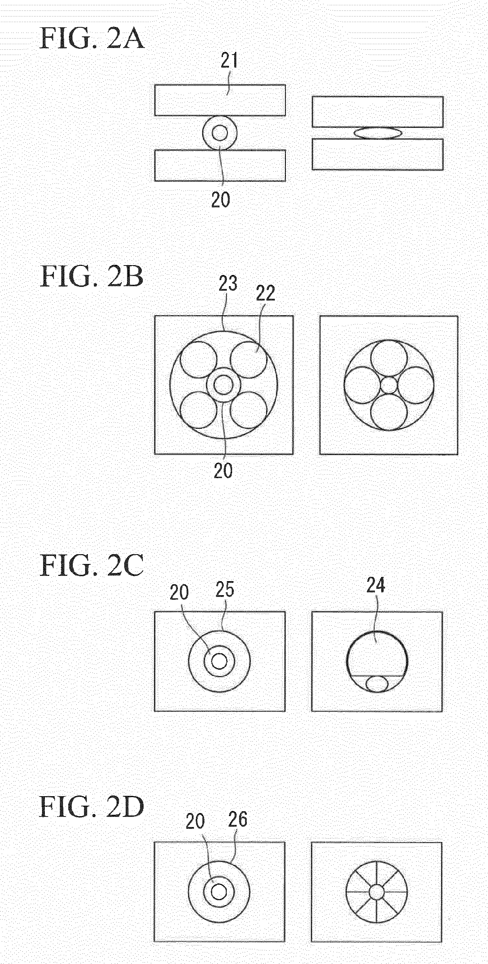

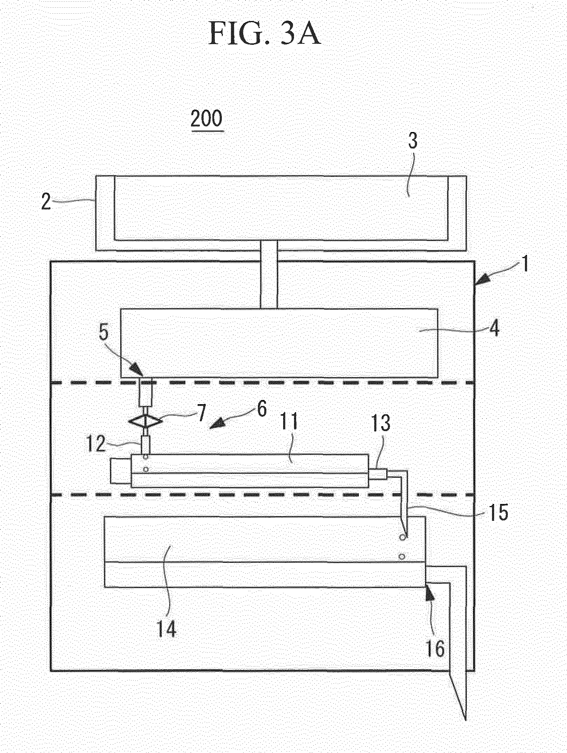

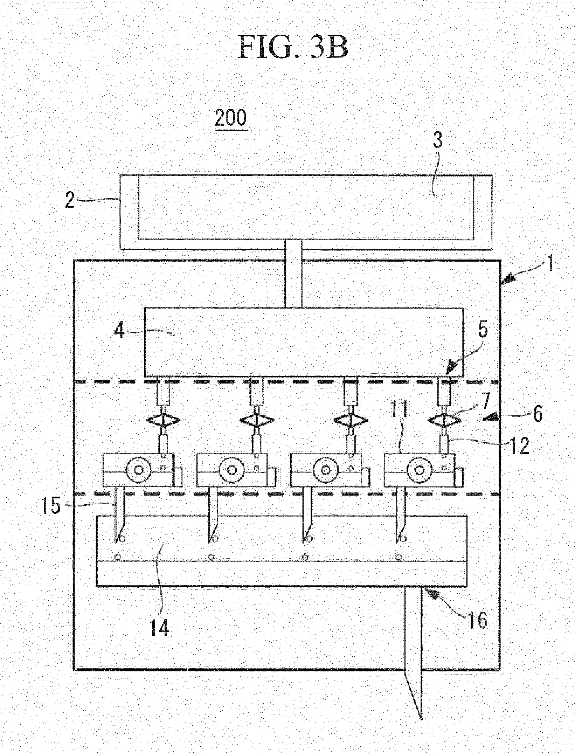

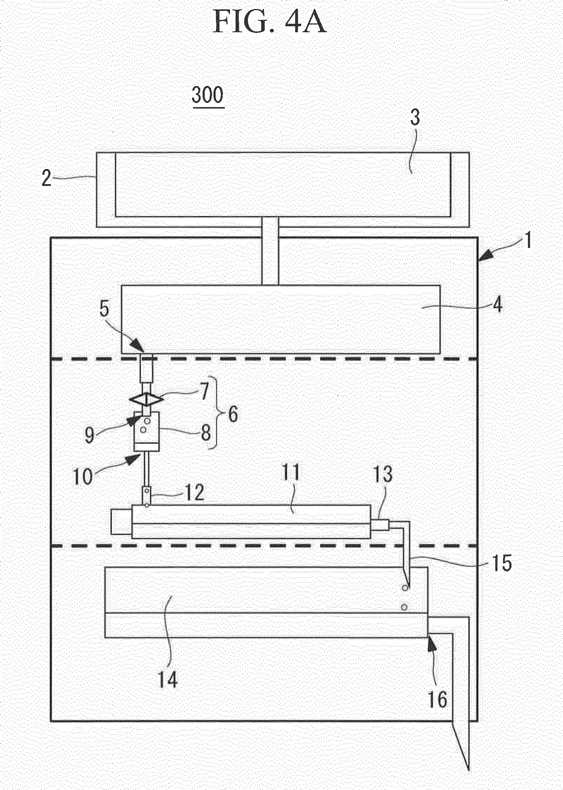

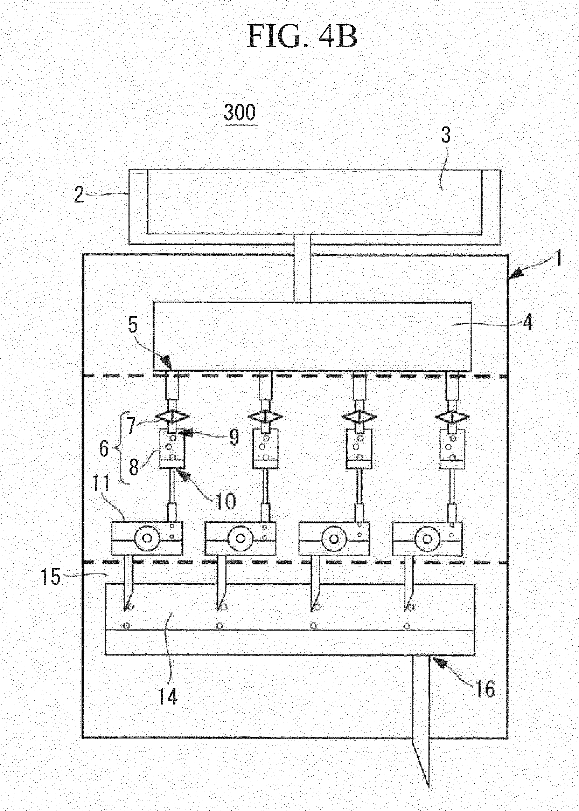

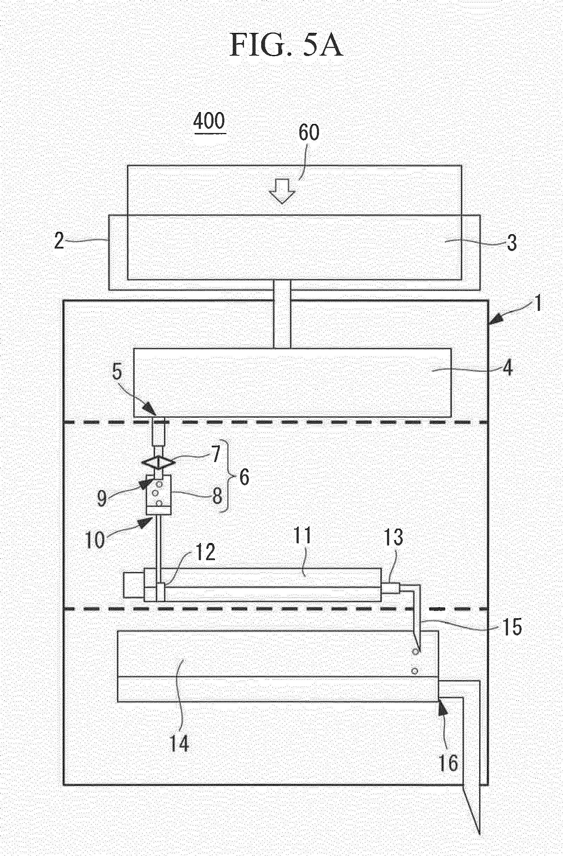

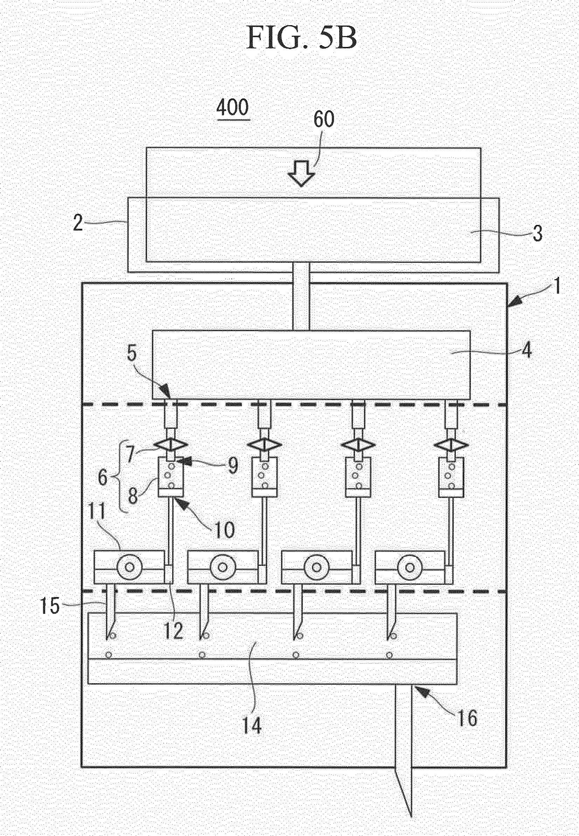

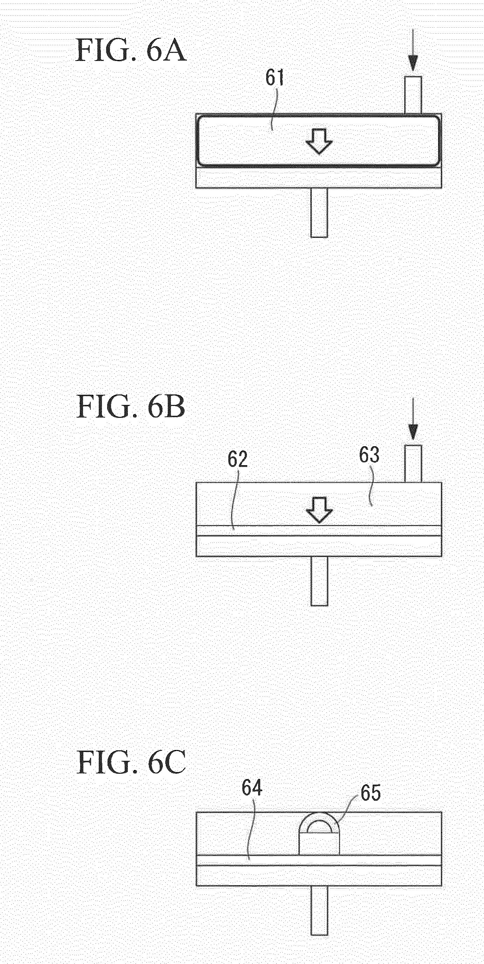

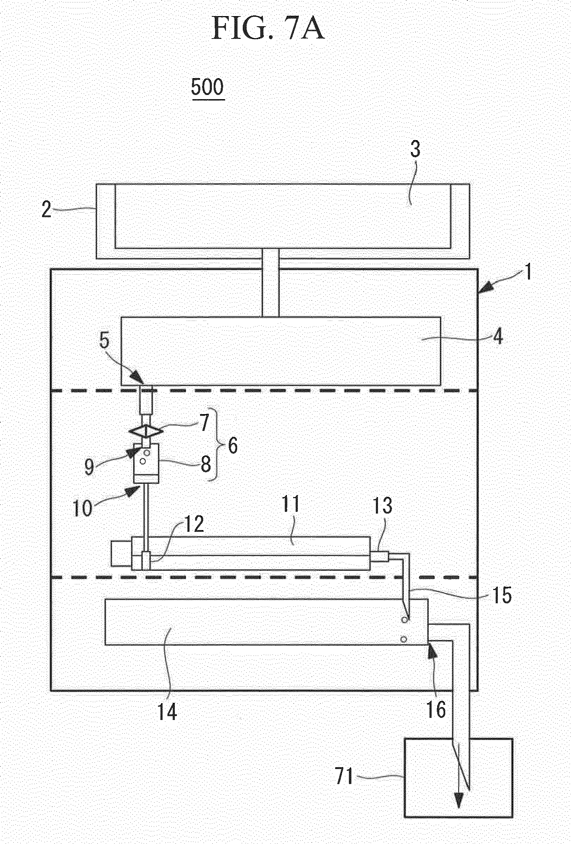

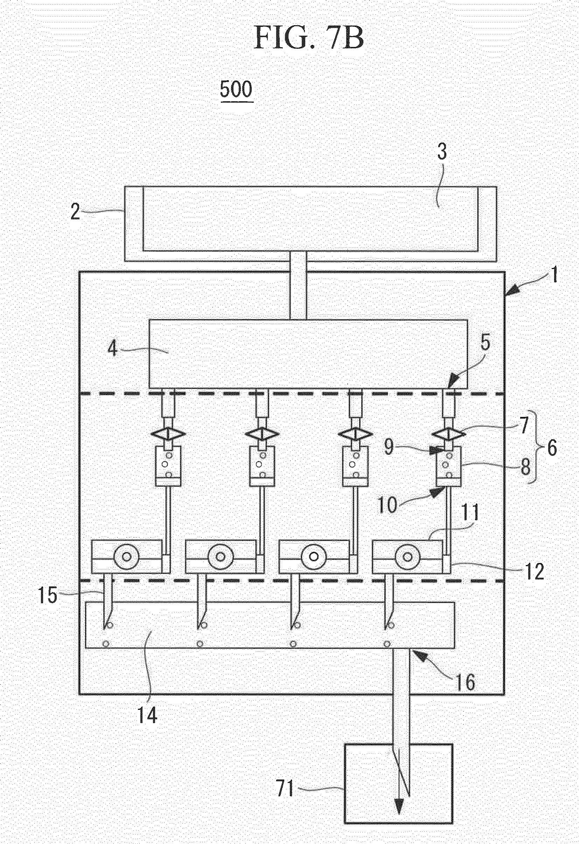

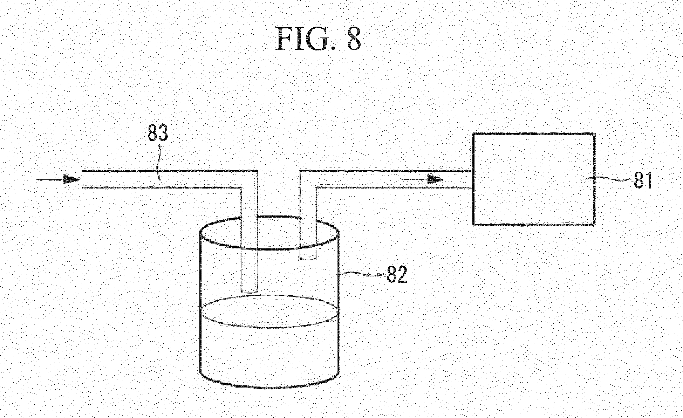

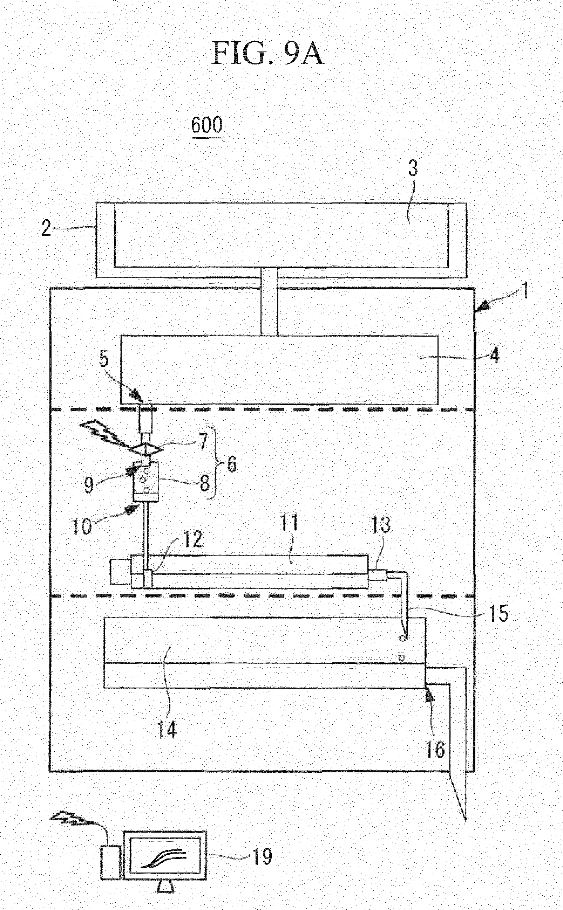

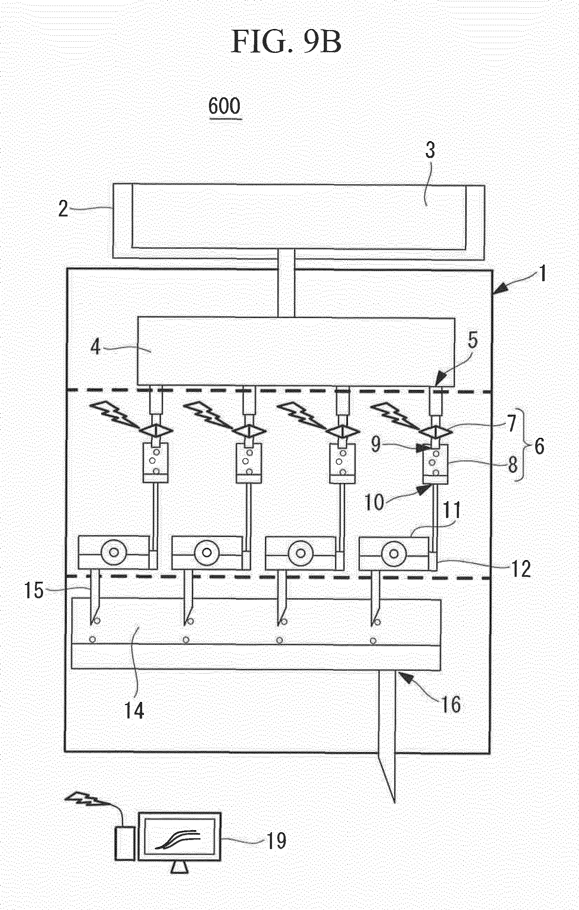

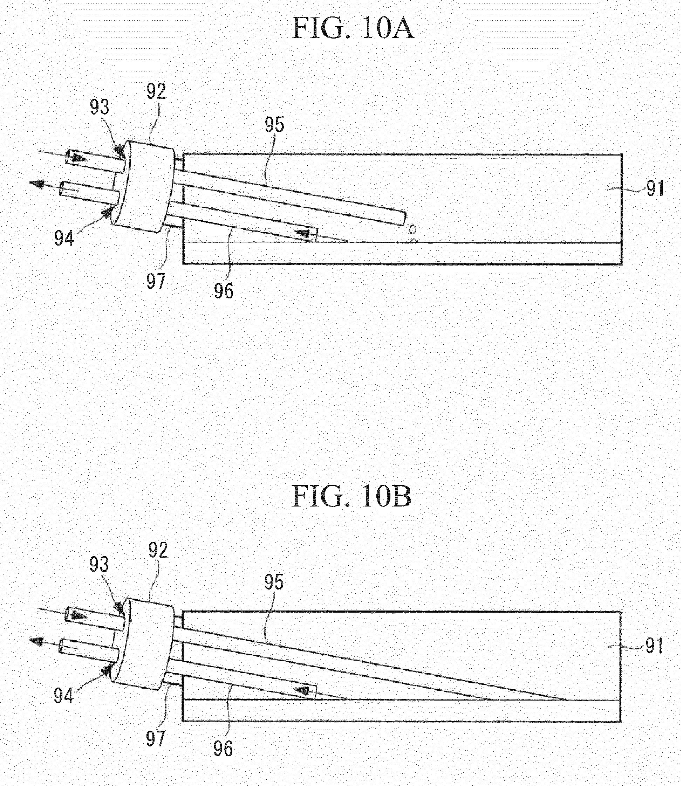

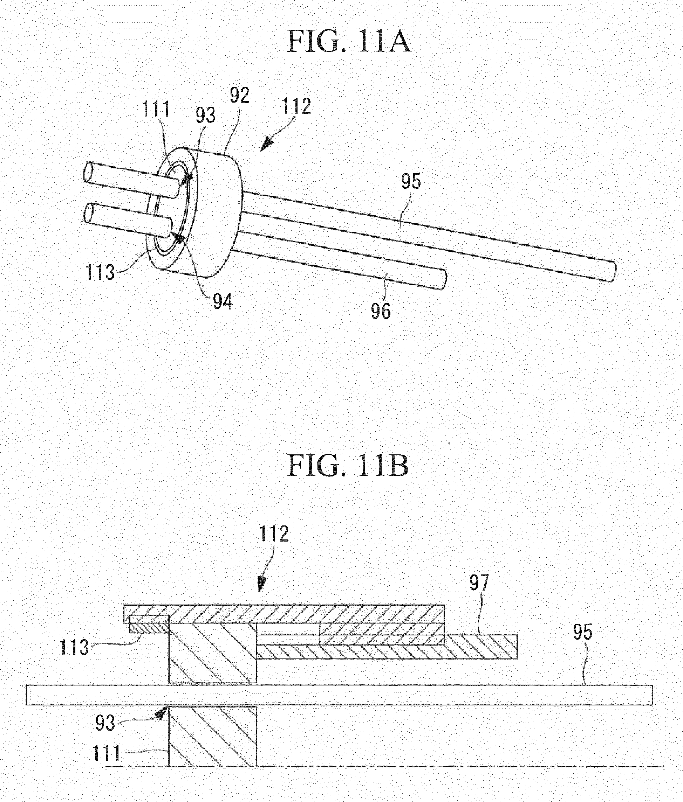

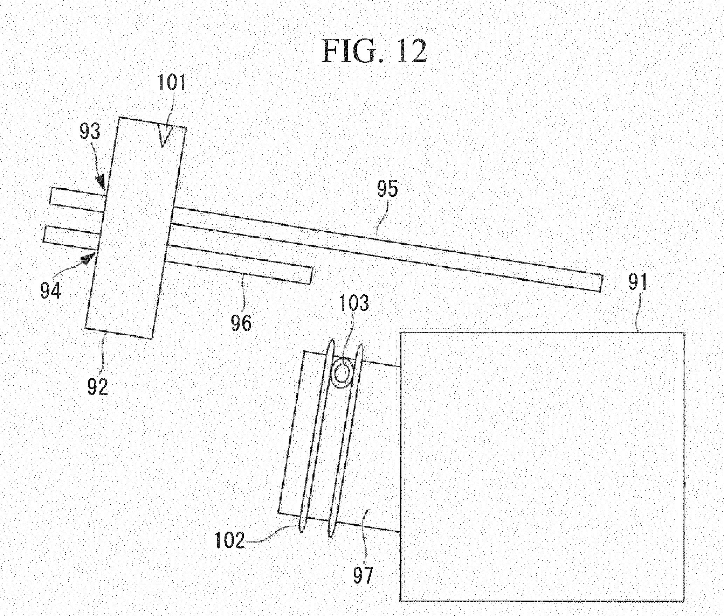

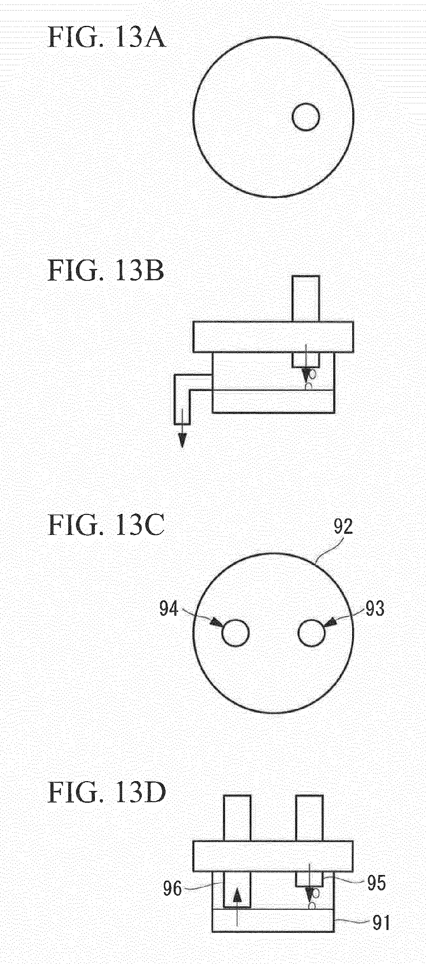

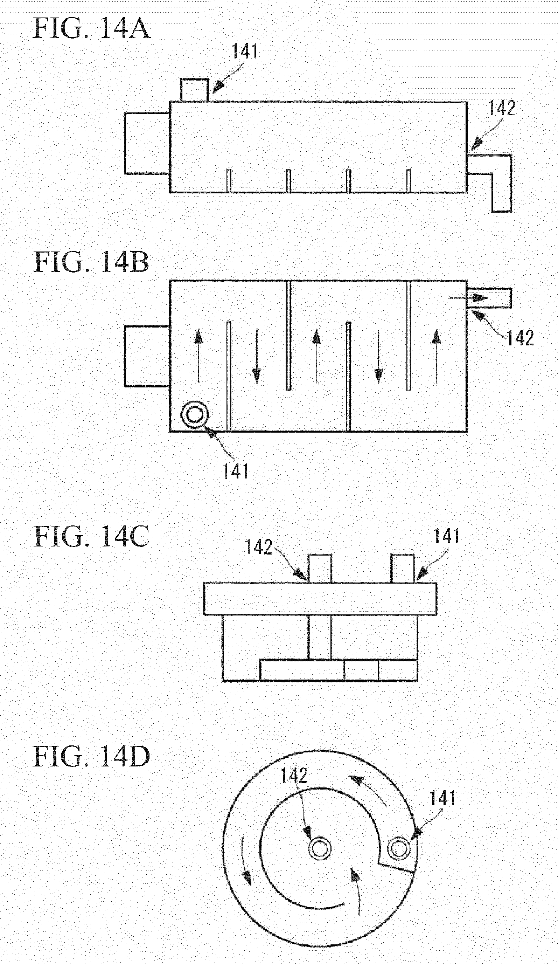

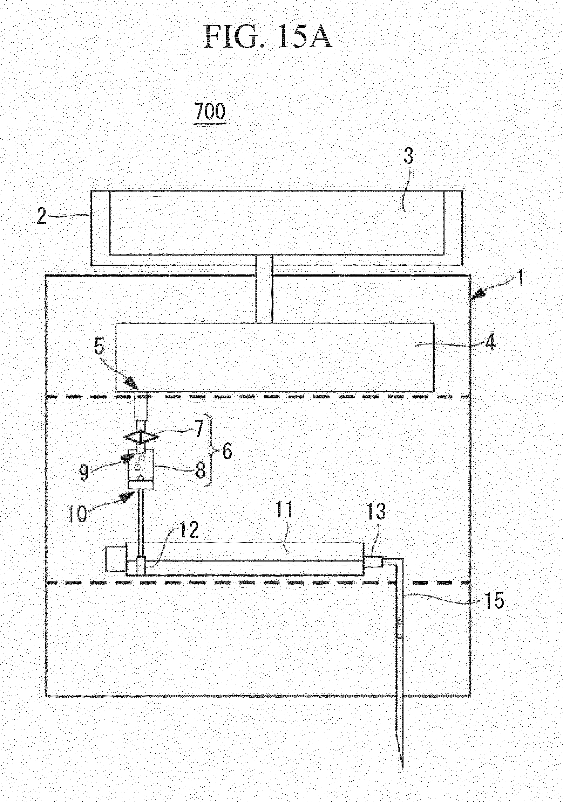

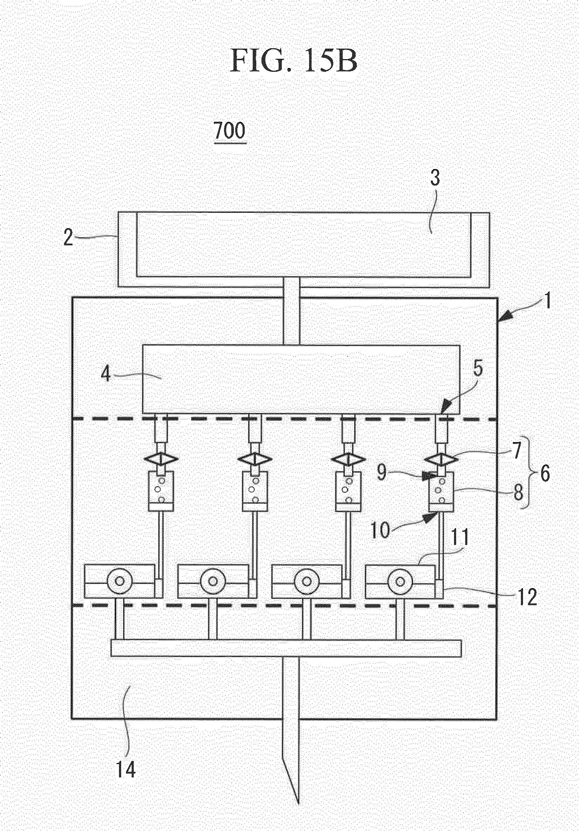

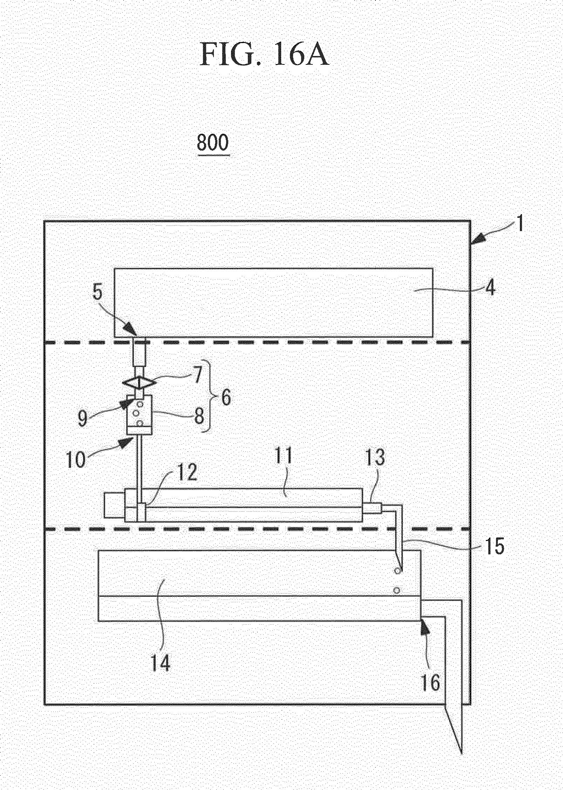

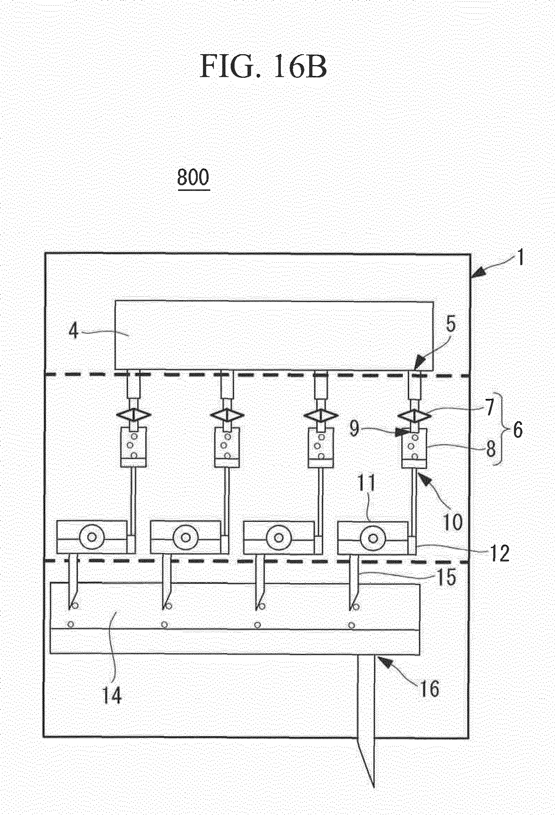

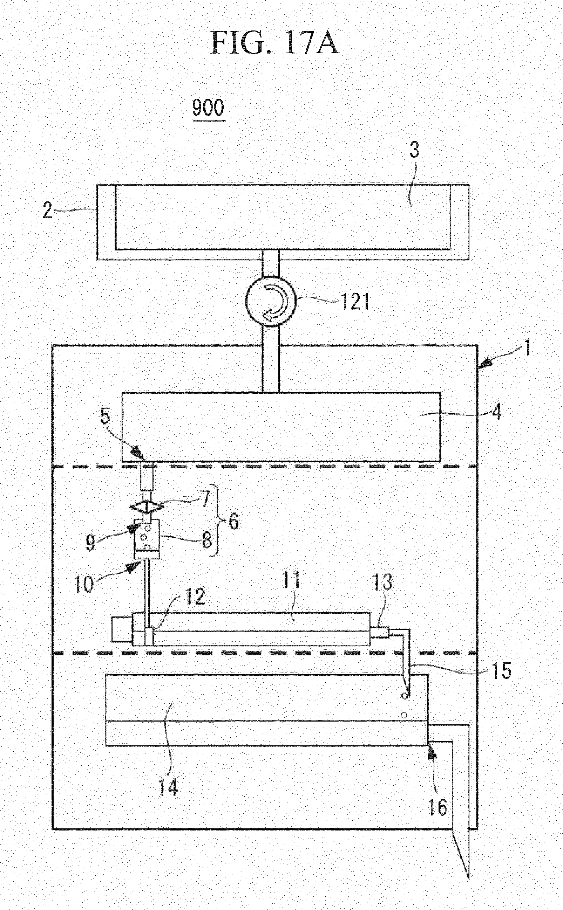

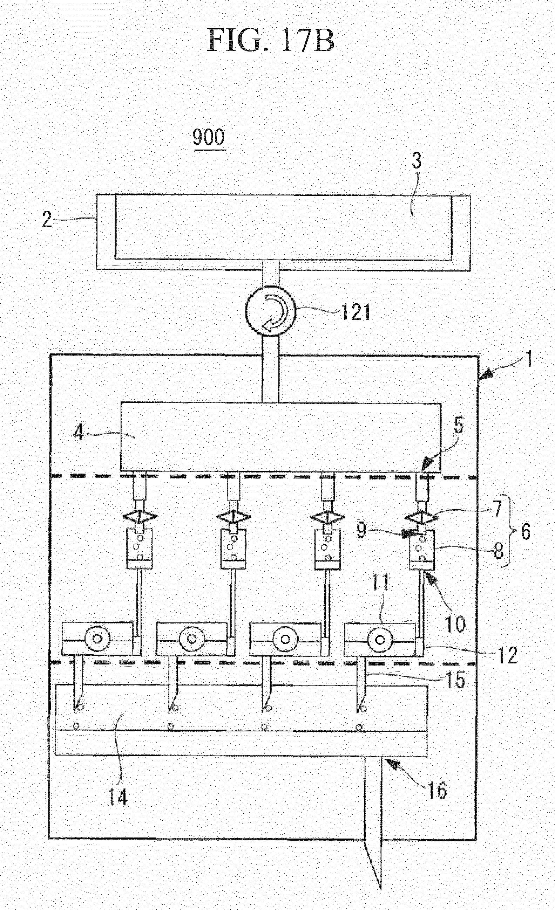

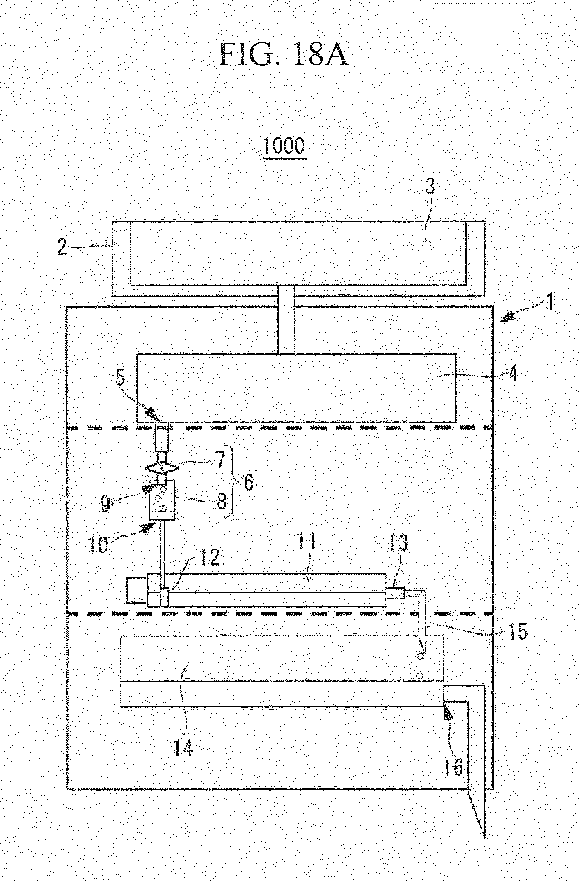

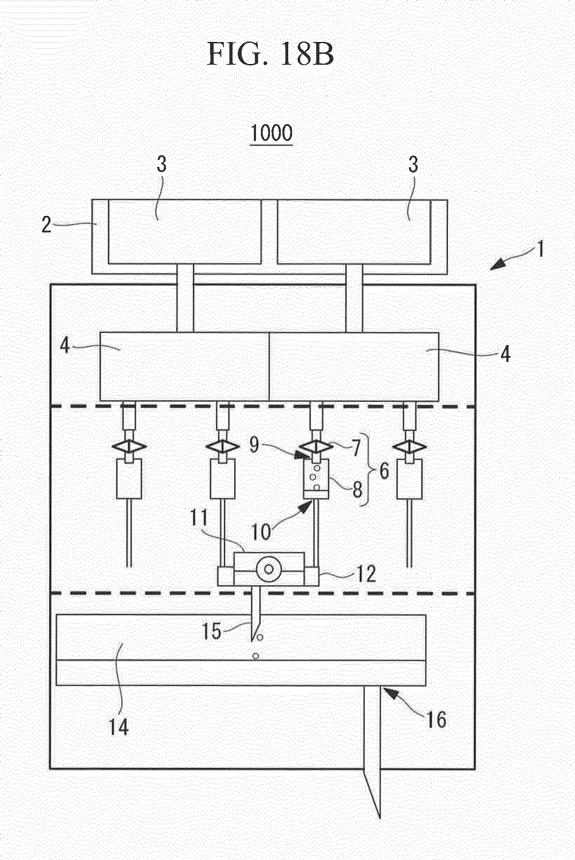

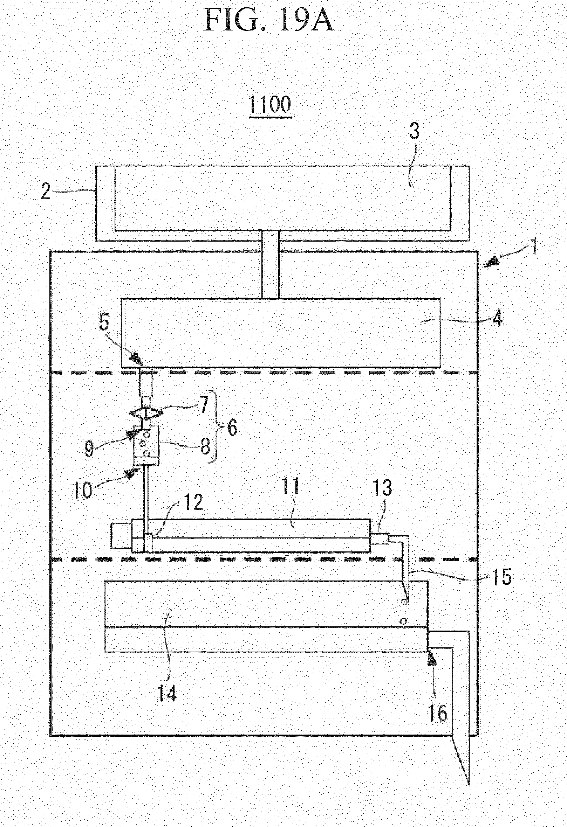

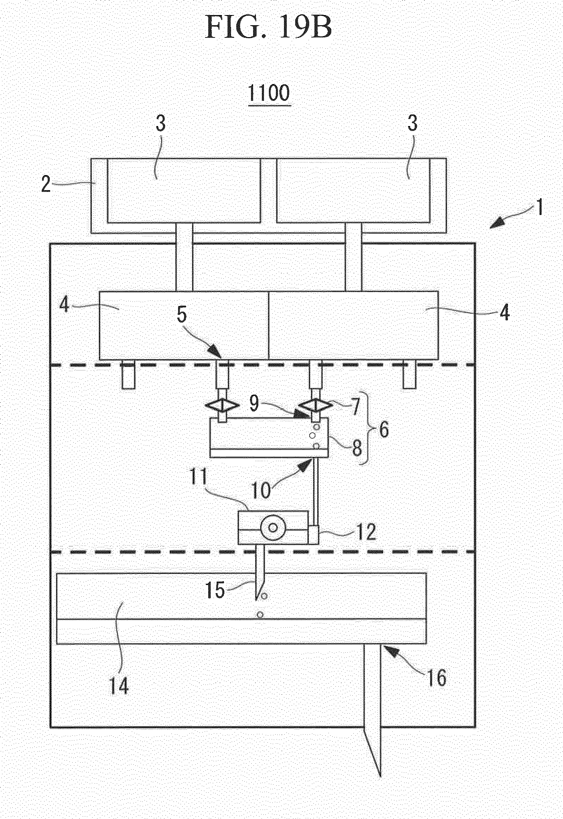

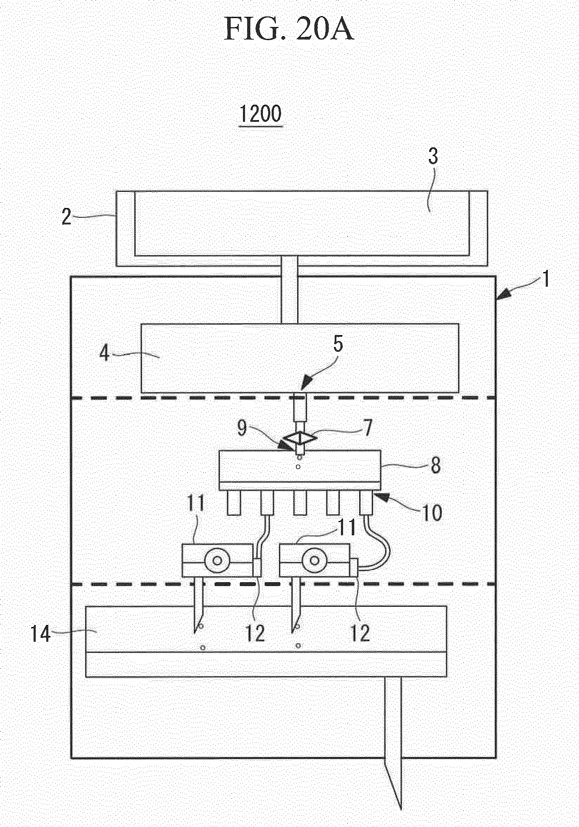

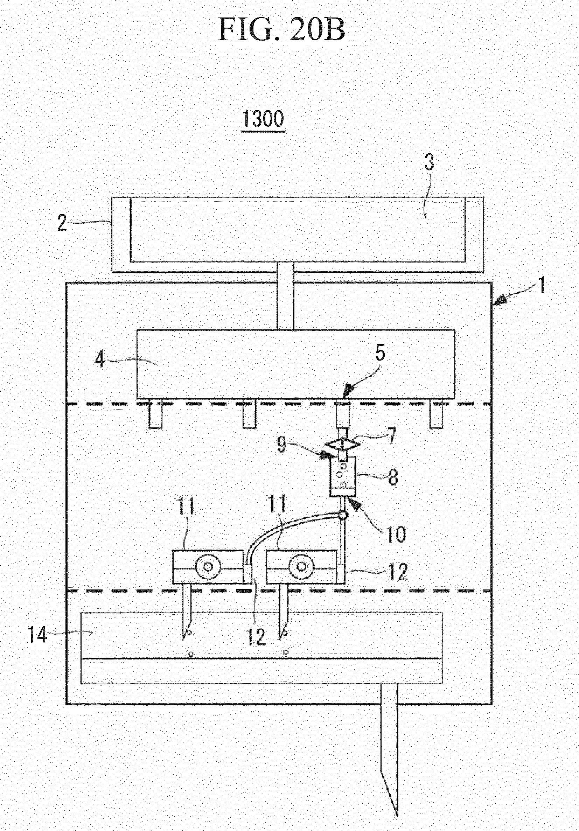

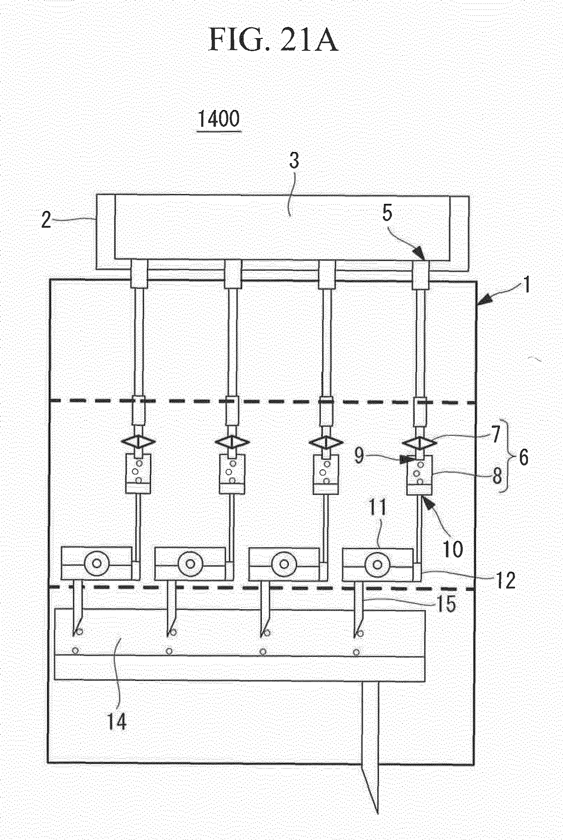

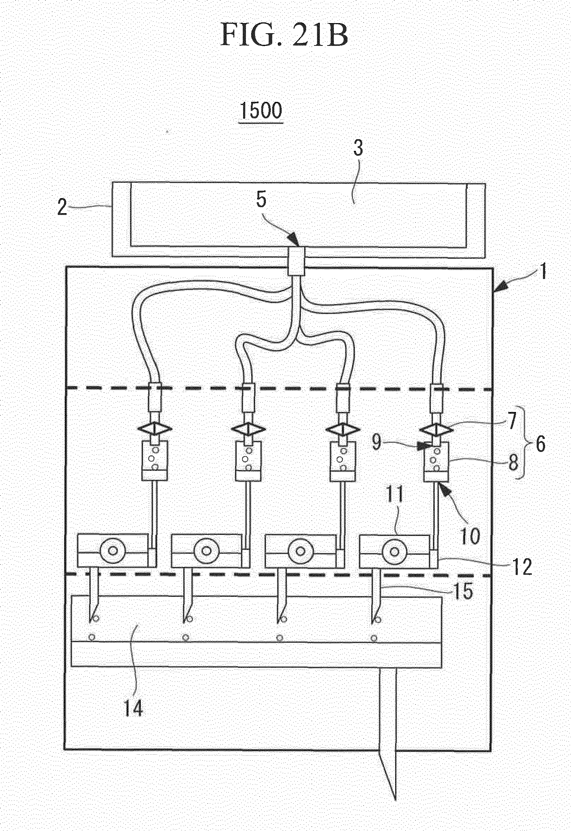

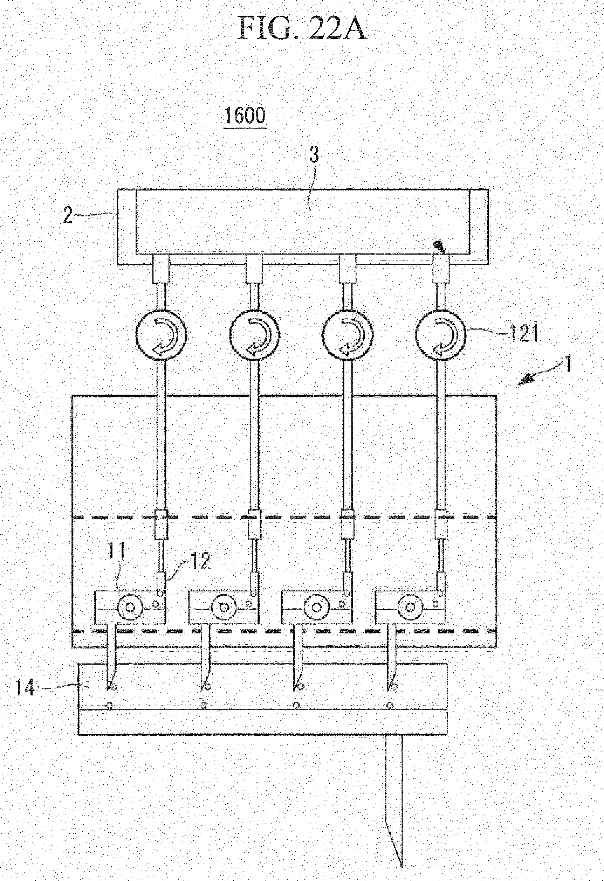

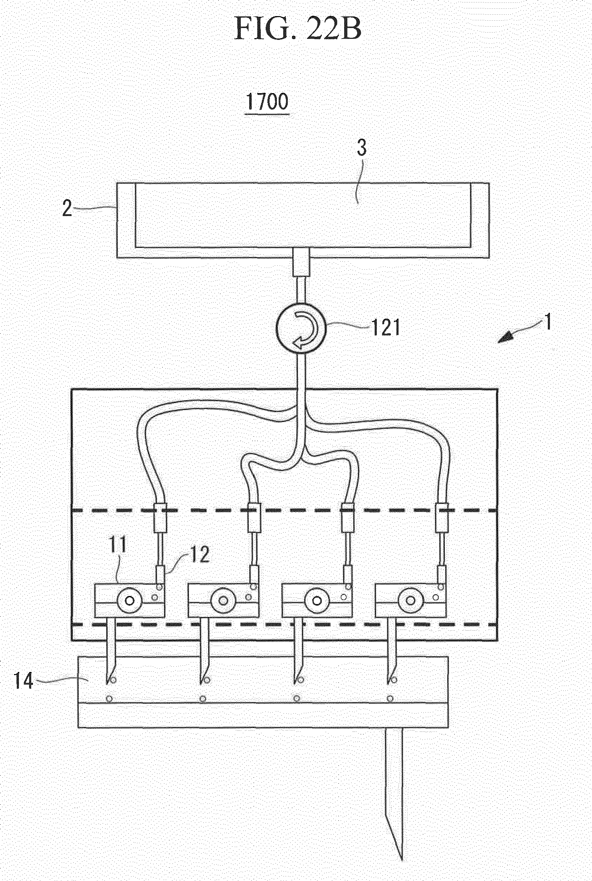

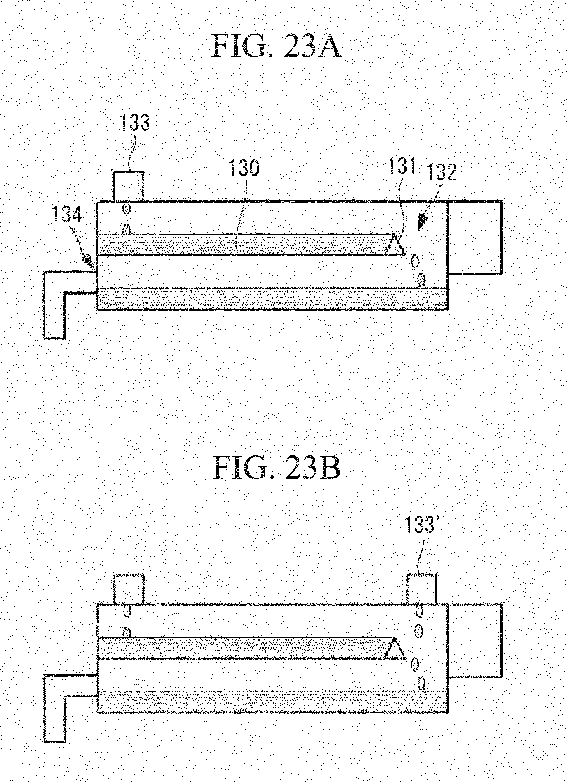

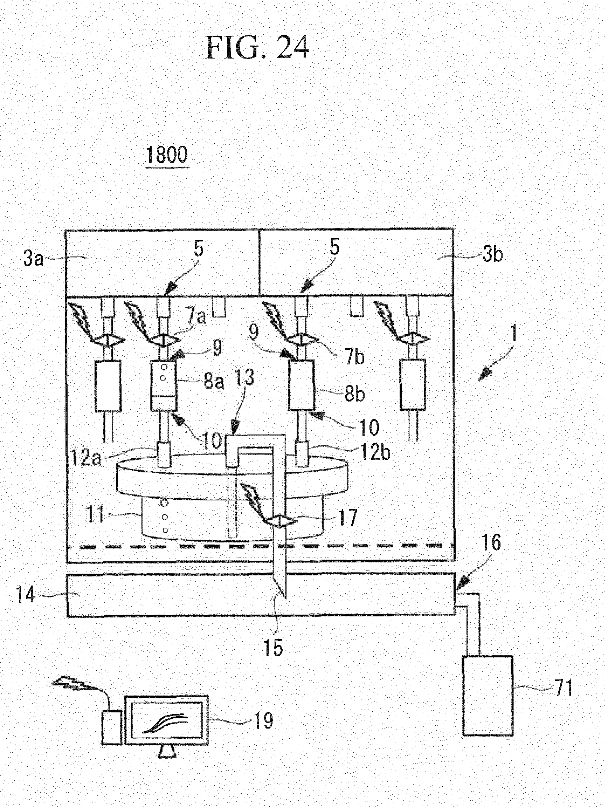

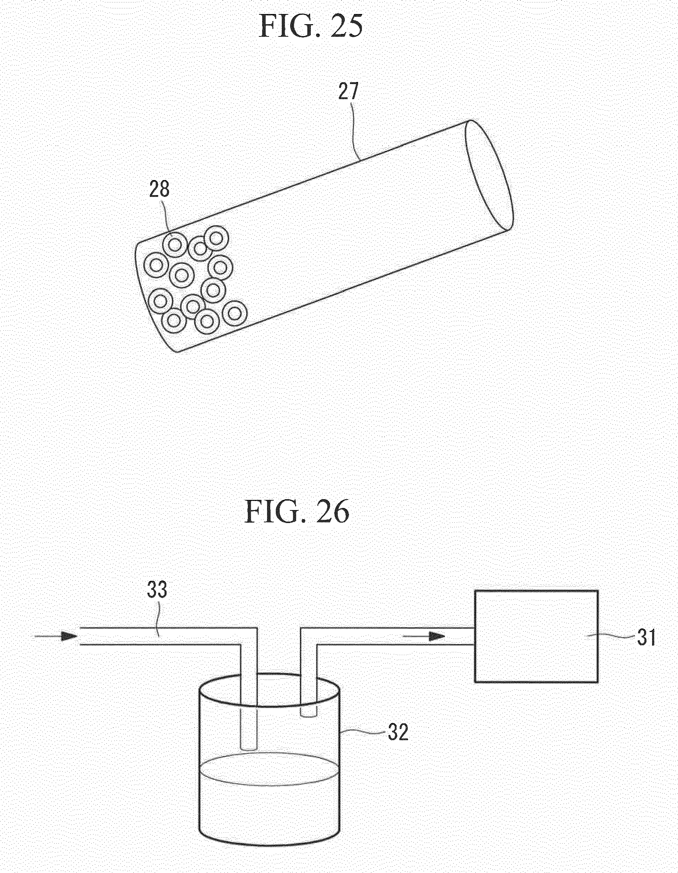

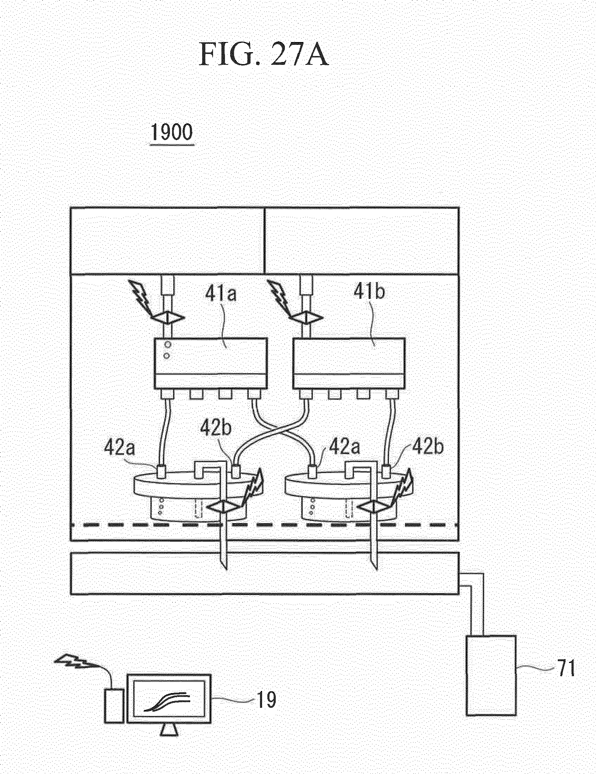

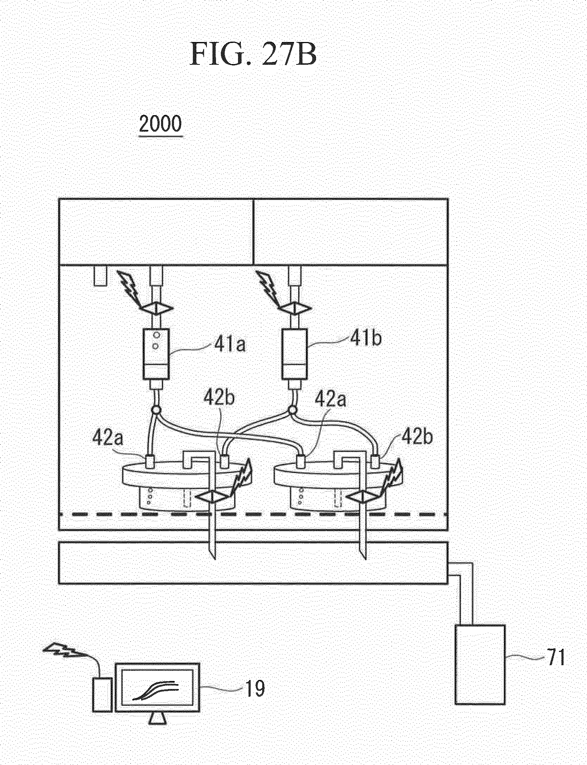

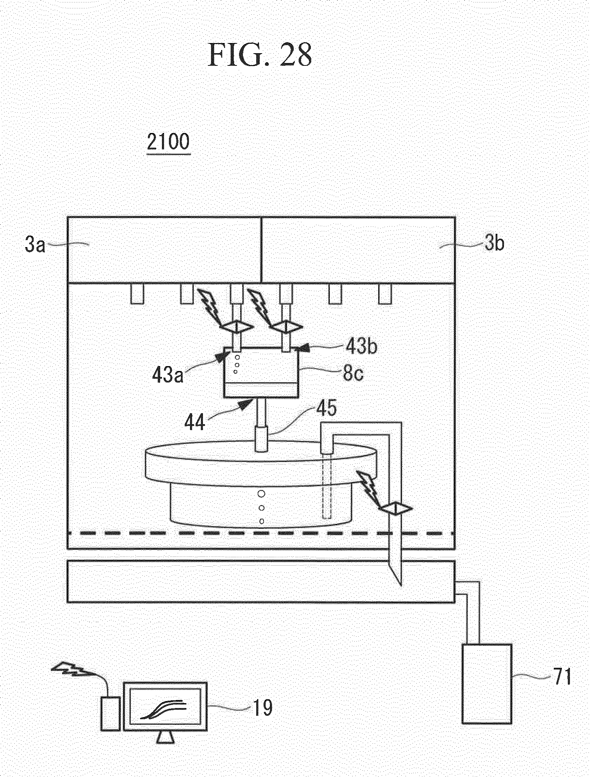

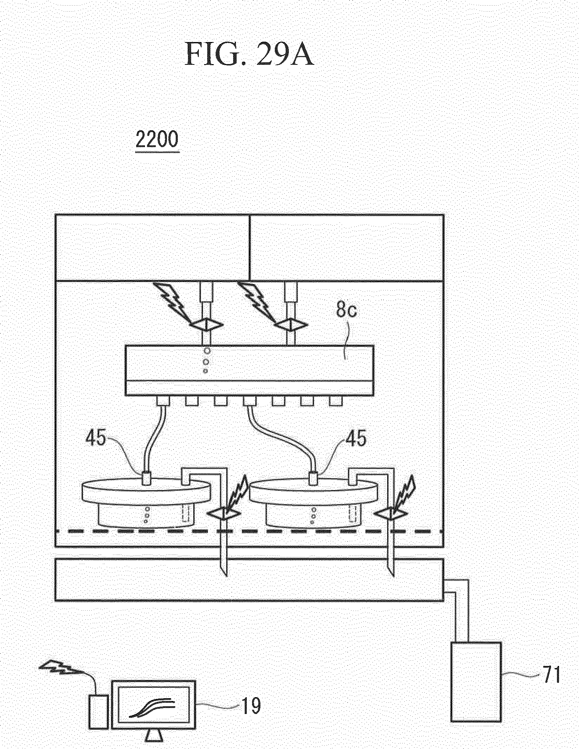

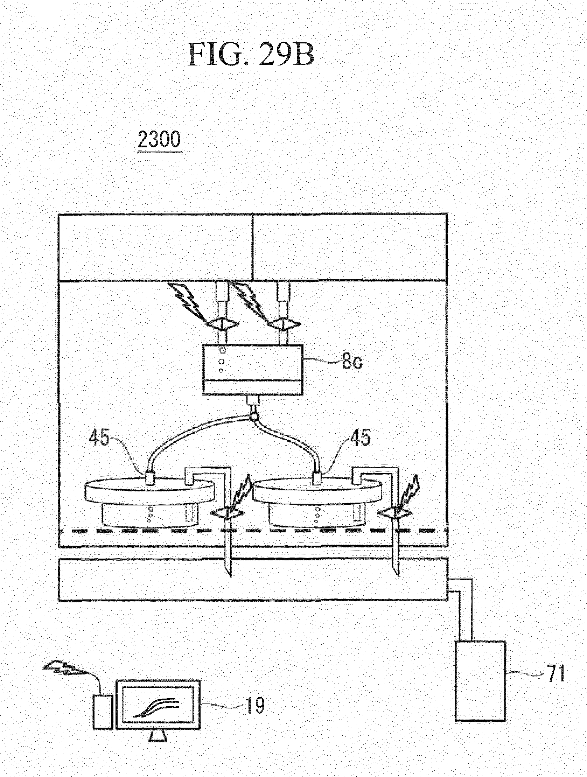

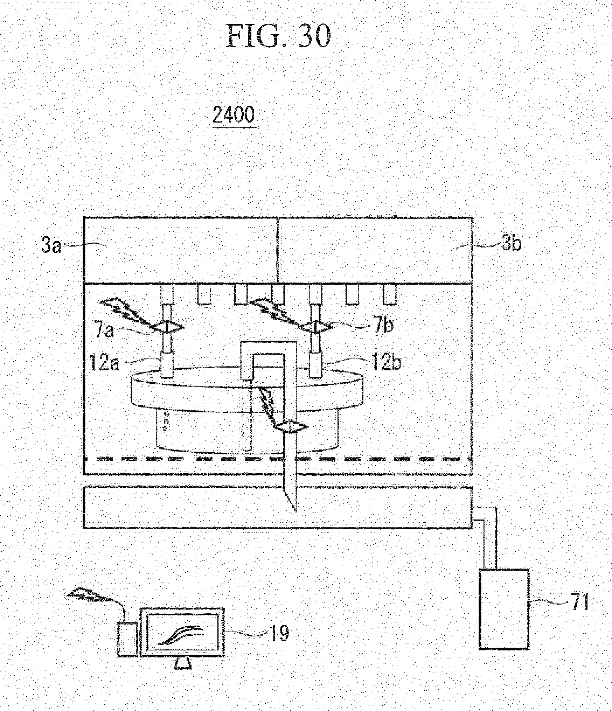

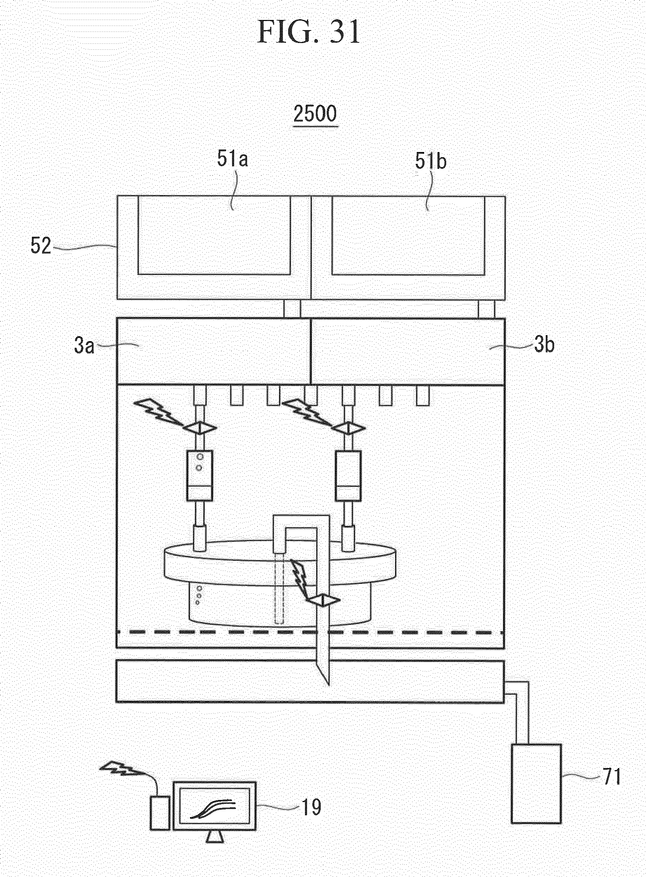

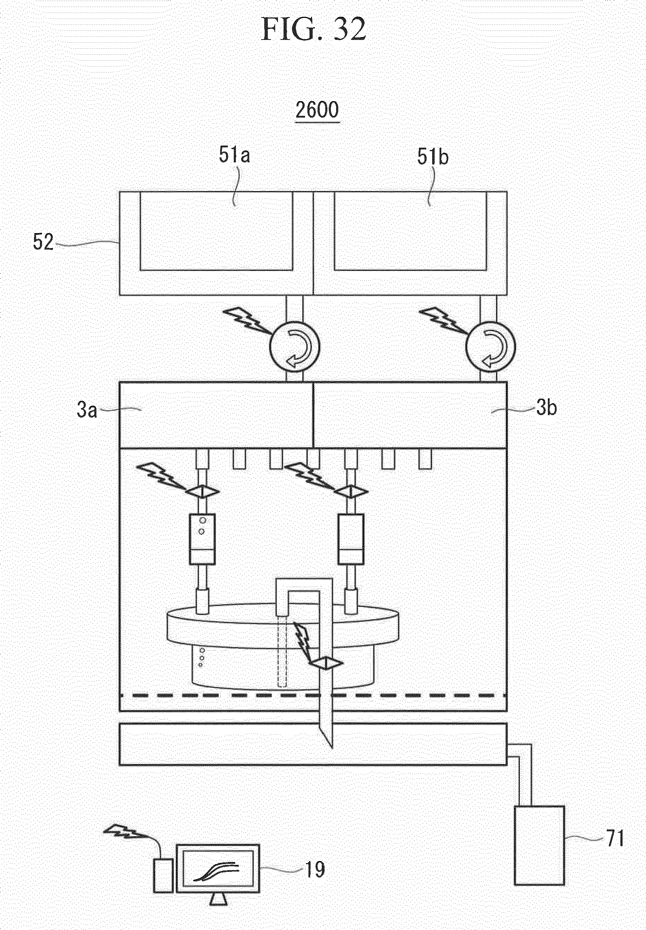

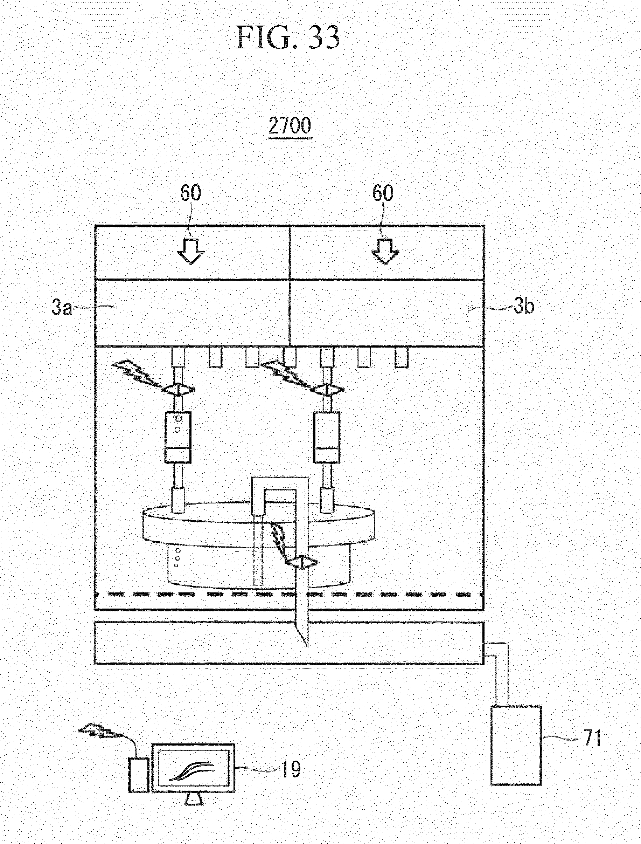

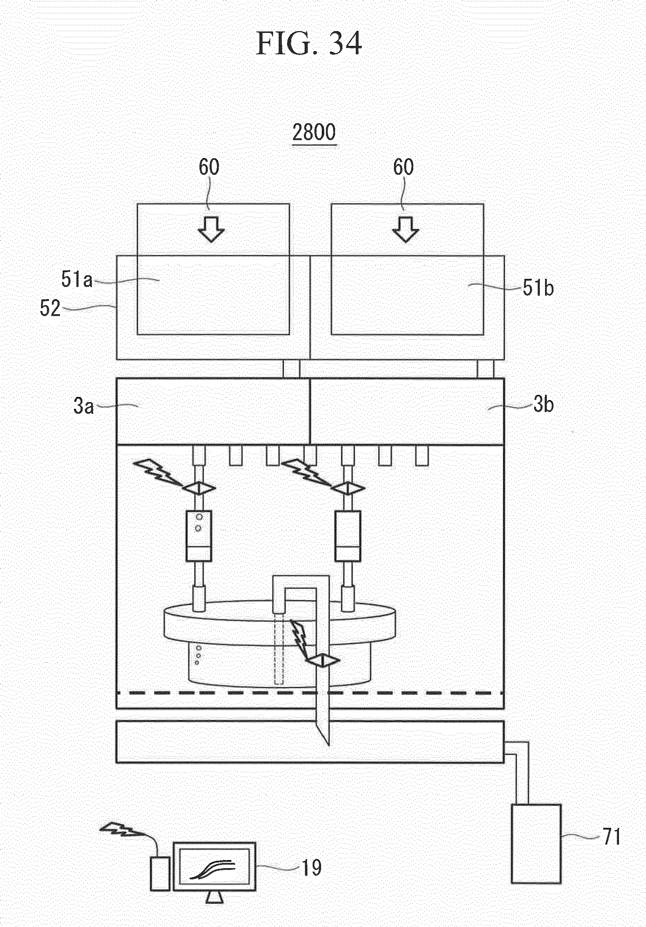

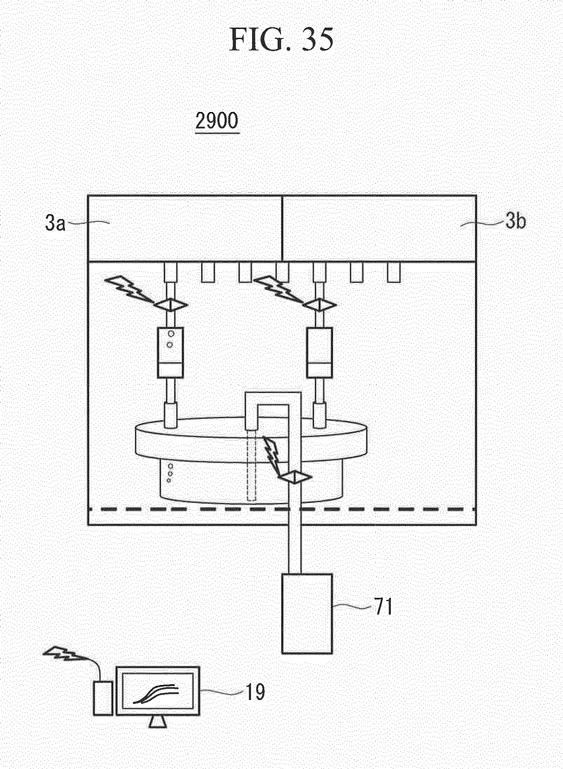

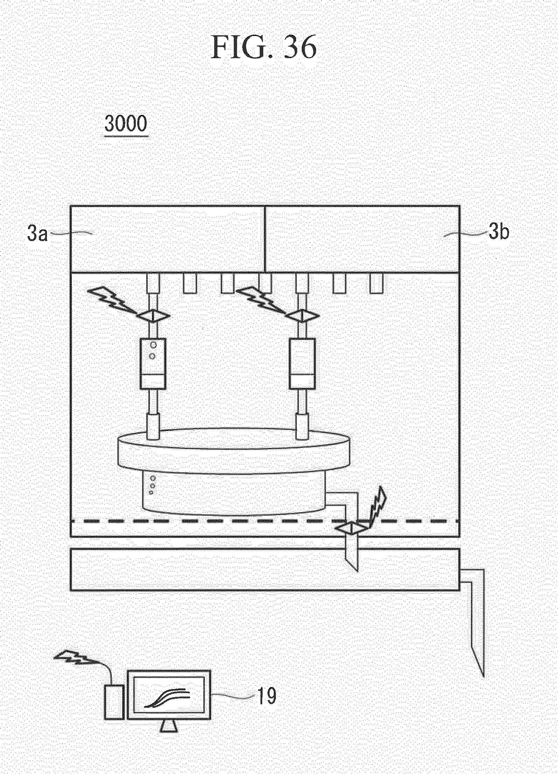

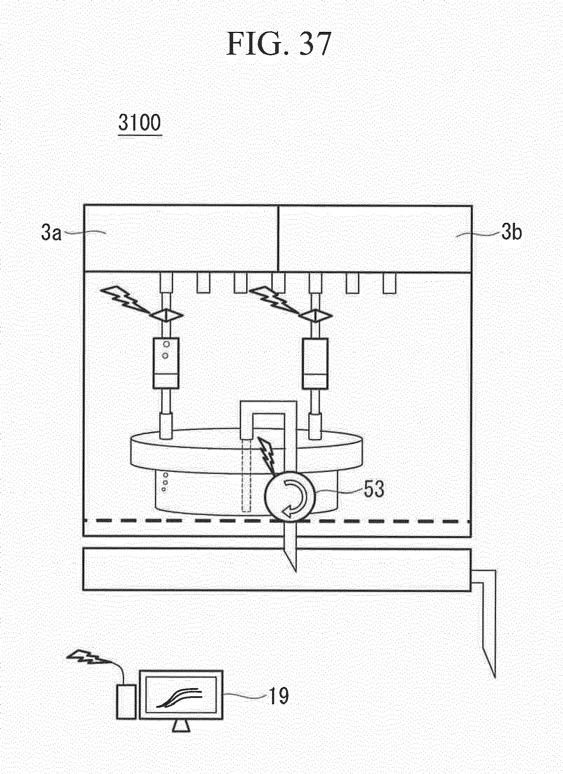

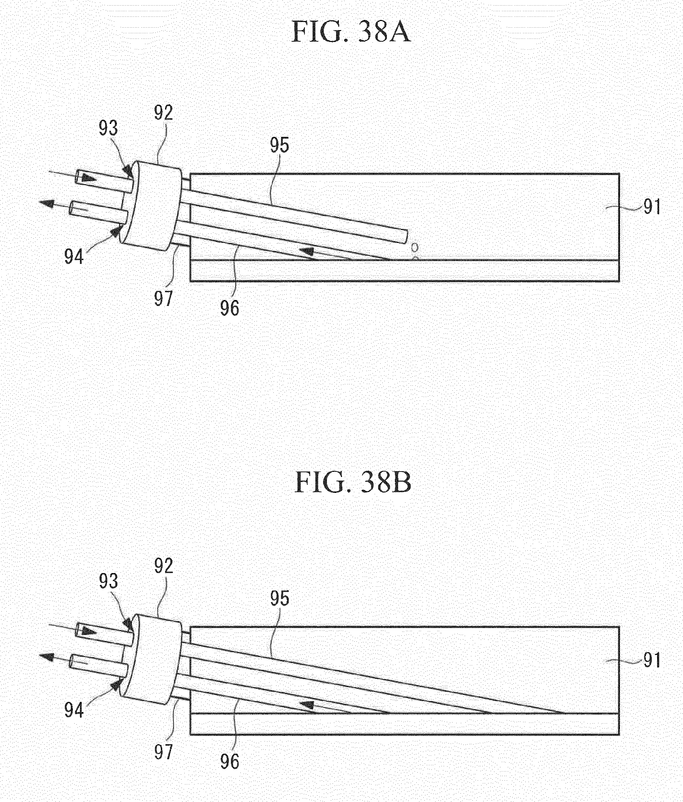

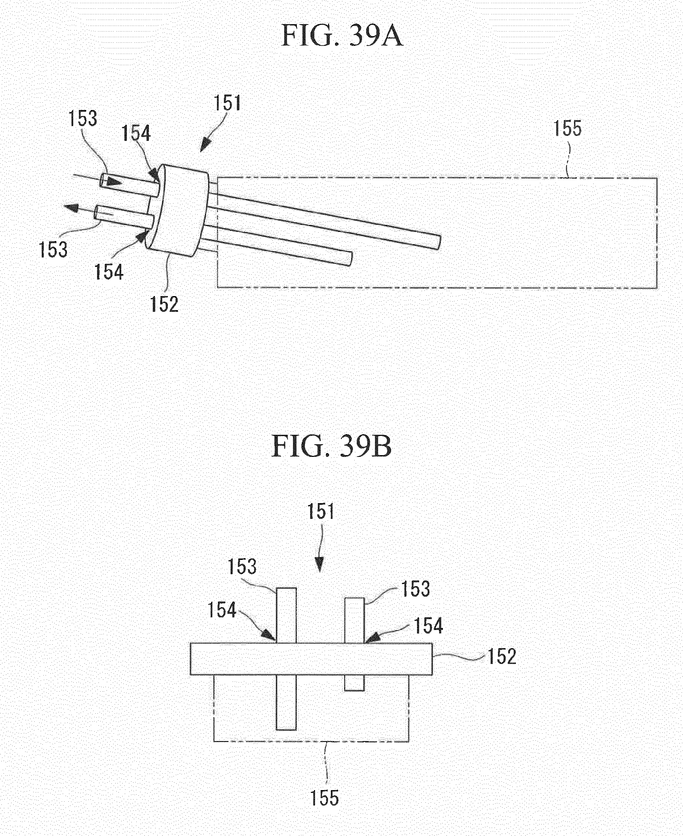

{Fig. 1A} Fig. 1A is a side view showing, in outline, the configuration of a cell-culturing system according to a first embodiment of the present invention.
{Fig. 1B} Fig. 1B is a front view showing, in outline, the configuration of the cell-culturing system according to the first embodiment of the present invention.
{Fig. 2A} Fig. 2A is a diagram for explaining, in outline, the configuration of an example of a dripping-speed adjusting means of the present invention.
{Fig. 2B} Fig. 2B is a diagram for explaining, in outline, the configuration of another example of the dripping-speed adjusting means of the present invention.
{Fig. 2C} Fig. 2C is a diagram for explaining, in outline, the configuration of yet another example of the dripping-speed adjusting means of the present invention.
{Fig. 2D} Fig. 2D is a diagram for explaining, in outline, the configuration of yet another example of the dripping-speed adjusting means of the present invention.
{Fig. 3A} Fig. 3A is a side view showing, in outline, the configuration of a cell-culturing system according to a second embodiment of the present invention.
{Fig. 3B} Fig. 3B is a front view showing, in outline, the configuration of the cell-culturing system according to the second embodiment of the present invention.
{Fig. 4A} Fig. 4A is a side view showing, in outline, the configuration of a cell-culturing system according to a modification of the second embodiment of the present invention.
{Fig. 4B} Fig. 4B is a front view showing, in outline, the configuration of the cell-culturing system according to the modification of the second embodiment of the present invention.
{Fig. 5A} Fig. 5A is a side view showing, in outline, the configuration of a cell-culturing system according to a third embodiment of the present invention.
{Fig. 5B} Fig. 5B is a front view showing, in outline, the configuration of the cell-culturing system according to the third embodiment of the present invention.
{Fig. 6A} Fig. 6A is a diagram for explaining, in outline, the configuration of an example of a pressurizing means of the present invention.
{Fig. 6B} Fig. 6B is a diagram for explaining, in outline, the configuration of another example of the pressurizing means of the present invention.
{Fig. 6C} Fig. 6C is a diagram for explaining, in outline, the configuration of yet another example of the pressurizing means of the present invention.
{Fig. 7A} Fig. 7A is a side view showing, in outline, the configuration of a cell-culturing system according to a fourth embodiment of the present invention.
{Fig. 7B} Fig. 7B is a front view showing, in outline, the configuration of the cell-culturing system according to the fourth embodiment of the present invention.
{Fig. 8} Fig. 8 is a diagram for explaining, in outline, the configuration of an example of a negative-pressure supplying means of the present invention.
{Fig. 9A} Fig. 9A is a side view showing, in outline, the configuration of a cell-culturing system according to a fifth embodiment of the present invention.
{Fig. 9B} Fig. 9B is a front view showing, in outline, the configuration of the cell-culturing system according to the fifth embodiment of the present invention.
{Fig. 10A} Fig. 10A is a diagram for explaining, in outline, the configuration of an example of a cell-culturing container that can be used in the present invention.
{Fig. 10B} Fig. 10B is a diagram for explaining, in outline, the configuration of another example of the cell-culturing container that can be used in the present invention.
{Fig. 11A} Fig. 11A is a perspective view showing an example of a lid portion of the cell-culturing container that can be used in the present invention.
{Fig. 11B} Fig. 11B is a partial sectional view showing, in outline, the configuration for a case in which the lid portion is attached to a neck portion of the cell-culturing container in an example of the cell-culturing container that can be used in the present invention.
{Fig. 12} Fig. 12 is a diagram for explaining, in outline, the configuration of an example of the cell-culturing container that can be used in the present invention.
{Fig. 13A} Fig. 13A is a plan view showing, in outline, the configuration of an example of the cell-culturing container that can be used in the present invention.
{Fig. 13B} Fig. 13B is a side view showing, in outline, the configuration of the example of the cell-culturing container that can be used in the present invention.
{Fig. 13C} Fig. 13C is a plan view showing, in outline, the configuration of another example of the cell-culturing container that can be used in the present invention.
{Fig. 13D} Fig. 13D is a side view showing, in outline, the configuration of another example of the cell-culturing container that can be used in the present invention.
{Fig. 14A} Fig. 14A is a side view showing, in outline, the configuration of an example of the cell-culturing container that can be used in the present invention.
{Fig. 14B} Fig. 14B is a plan view showing, in outline, the configuration of the example of the cell-culturing container that can be used in the present invention.
{Fig. 14C} Fig. 14C is a side view showing, in outline, the configuration of another example of the cell-culturing container that can be used in the present invention.
{Fig. 14D} Fig. 14D is a plan view showing, in outline, the configuration of the example of the cell-culturing container that can be used in the present invention.
{Fig. 15A} Fig. 15A is a side view showing, in outline, the configuration of a modification of the individual embodiments of the present invention.
{Fig. 15B} Fig. 15B is a front view showing, in outline, the configuration of the modification of the individual embodiments of the present invention.
{Fig. 16A} Fig. 16A is a side view showing, in outline, the configuration of a modification of the individual embodiments of the present invention.
{Fig. 16B} Fig. 16B is a front view showing, in outline, the configuration of the modification of the individual embodiments of the present invention.
{Fig. 17A} Fig. 17A is a side view showing, in outline, the configuration of a modification of the individual embodiments of the present invention.
{Fig. 17B} Fig. 17B is a front view showing, in outline, the configuration of the modification of the individual embodiments of the present invention.
{Fig. 18A} Fig. 18A is a side view showing, in outline, the configuration of a modification of the individual embodiments of the present invention.
{Fig. 18B} Fig. 18B is a front view showing, in outline, the configuration of the modification of the individual embodiments of the present invention.
{Fig. 19A} Fig. 19A is a side view showing, in outline, the configuration of a modification of the individual embodiments of the present invention.
{Fig. 19B} Fig. 19B is a front view showing, in outline, the configuration of the modification of the individual embodiments of the present invention.
{Fig. 20A} Fig. 20A is a front view showing, in outline, the configuration of a modification of the individual embodiments of the present invention.
{Fig. 20B} Fig. 20B is a front view showing, in outline, the configuration of another modification of the individual embodiments of the present invention.
{Fig. 21A} Fig. 21A is a front view showing, in outline, the configuration of a modification of the individual embodiments of the present invention.
{Fig. 21B} Fig. 21B is a front view showing, in outline, the configuration of another modification of the individual embodiments of the present invention
{Fig. 22A} Fig. 22A is a front view showing, in outline, the configuration of a modification of the individual embodiments of the present invention.
{Fig. 22B} Fig. 22B is a front view showing, in outline, the configuration of another modification of the individual embodiments of the present invention.
{Fig. 23A} Fig. 23A is a side view showing, in outline, the configuration of an example of the cell-culturing container that can be used in the present invention.
{Fig. 23B} Fig. 23B is a side view showing, in outline, the configuration of another example of the cell-culturing container that can be used in the present invention.
{Fig. 24} Fig. 24 is a front view showing, in outline, the configuration of a cell-culturing system according to a sixth embodiment of the present invention.
{Fig. 25} Fig. 25 is a diagram for explaining, in outline, the configuration of an example of a tubular member protruding into the cell-culturing container of the present invention.
{Fig. 26} Fig. 26 is a diagram for explaining, in outline, the configuration of an example of a negative-pressure supplying means of the present invention.
{Fig. 27A} Fig. 27A is a front view showing, in outline, the configuration of a cell-culturing system according to a modification of the sixth embodiment of the present invention.
{Fig. 27B} Fig. 27B is a front view showing, in outline, the configuration of a cell-culturing system according to another modification of the sixth embodiment of the present invention.
{Fig. 28} Fig. 28 is a front view showing, in outline, the configuration of a cell-culturing system according to a modification of the sixth embodiment of the present invention.
{Fig. 29A} Fig. 29A is a front view showing, in outline, the configuration of a cell-culturing system according to a modification of the sixth embodiment of the present invention.
{Fig. 29B} Fig. 29B is a front view showing, in outline, the configuration of a cell-culturing system according to another modification of the sixth embodiment of the present invention.
{Fig. 30} Fig. 30 is a front view showing, in outline, the configuration of a cell-culturing system according to a seventh embodiment of the present invention.
{Fig. 31} Fig. 31 is a front view showing, in outline, the configuration of a cell-culturing system according to a modification of the individual embodiments of the present invention.
{Fig. 32} Fig. 32 is a front view showing, in outline, the configuration of a cell-culturing system according to a modification of the individual embodiments of the present invention.
{Fig. 33} Fig. 33 is a front view showing, in outline, the configuration of a cell-culturing system according to a modification of the individual embodiments of the present invention.
{Fig. 34} Fig. 34 is a front view showing, in outline, the configuration of a cell-culturing system according to a modification of the individual embodiments of the present invention.
{Fig. 35} Fig. 35 is a front view showing, in outline, the configuration of a cell-culturing system according to a modification of the individual embodiments of the present invention.
{Fig. 36} Fig. 36 is a front view showing, in outline, the configuration of a cell-culturing system according to a modification of the individual embodiments of the present invention.
{Fig. 37} Fig. 37 is a front view showing, in outline, the configuration of a cell-culturing system according to a modification of the individual embodiments of the present invention.
{Fig. 38A} Fig. 38A is a side view showing, in outline, the configuration of an example of the cell-culturing container that can be used in the present invention.
{Fig. 38B} Fig. 38B is a side view showing, in outline, the configuration of another example of the cell-culturing container that can be used in the present invention.
{Fig. 39A} Fig. 39A is a perspective view showing, in outline, the configuration of an example of a lid portion of the cell-culturing container that can be used in the present invention.
{Fig. 39B} Fig. 39B is a side view showing, in outline, the configuration of another example of the lid portion of the cell-culturing container that can be used in the present invention.

### {Description of Embodiments}

Cell-culturing systems according to embodiments of the present invention will be described below with reference to the drawings.

### {First Embodiment}

A cell-culturing system 100 according to this embodiment is a system that is configured as shown in Figs. 1A and 1B, and is a system in which a medium in a cell-culturing container (culture vessel) in an incubator is replaced. Fig. 1A shows a side view thereof, and Fig. 1B shows a front view thereof.

A medium-preserving means 3 is installed outside an incubator 1, and preserves the medium (cell-culturing liquid) in the interior thereof. A temperature-controlling means 2 is provided in order to maintain the temperature of the medium at an appropriate temperature (for example, 4 °C) for preservation thereof.

The medium-preserving means 3 is connected to a temporary holding means 4 that is installed in the interior of the incubator 1 via a tubular member (tube or the like). Because the medium-preserving means 3 is installed at a position that is higher than the temporary holding means 4 in the direction in which gravity acts, the medium inside the medium-preserving means 3 is supplied to the temporary holding means 4 via the tubular member (tube or the like) by the action of gravity. The medium that has been supplied to the temporary holding means 4 is warmed so as to reach the temperature in the incubator, which is an appropriate temperature for cell culturing (for example, 37 °C).

The medium that has been warmed, by means of the temporary holding means 4, to the appropriate temperature for cell culturing (for example, 37 °C) is supplied to a cell-culturing container 11 in the incubator 1 via a medium-supplying means 6.

The medium-supplying means 6 is provided with a dripping-speed adjusting means 7 and a dripping container 8. The dripping-speed adjusting means 7 is installed in a tubular member that connects the temporary holding means 4 and the dripping container 8, and is capable of controlling the flow speed of the medium that flows inside the tubular member.

The dripping container 8 is a container having a supply port 9 that is connected to the tubular member leading thereto from the temporary holding means 4 and a discharging port 10 that is connected to a tubular member leading to a cell-culturing container 11. The supply port 9 is disposed at an upper portion in the direction in which gravity acts, and the discharging port 10 is disposed at a portion that is lower than the supply port 9 in the direction in which gravity acts. The medium that has been supplied to the supply port 9 is made to drip in a space inside the dripping container 8, is discharged from the discharging port 10, and is supplied to the cell-culturing container 11 via the tubular member.

As has been described above, by making the medium drip in the space in the dripping container 8, it is possible to prevent a reverse flow of the medium, and thus, it is possible to prevent the medium in the temporary holding means 4 and the medium-preserving means 3 from becoming polluted (the occurrence of contamination).

The cell-culturing container 11 has a supply port 12 that is connected to the tubular member leading thereto from the dripping container 8, and a discharging port 13 through which the medium is discharged outside the cell-culturing container. The discharging port 13 is disposed in a side surface of the cell-culturing container 11 and is configured so as to discharge the medium to a waste-liquid holding means 14 through the discharging port 13 when the amount of medium (height of the medium) in the cell-culturing container exceeds a certain value. The amount of medium held in the cell-culturing container is determined by the height at which the discharging port 13 is installed. On the other hand, although the position at which the supply port 12 is installed is arbitrary, it is possible to increase the conversion efficiency of the medium by increasing the distance from the discharging port 13 as much as possible.

The waste-liquid holding means 14 has a waste-liquid supply port 15 that is connected to a tubular member leading thereto from the cell-culturing container 11 and a waste-liquid discharging port 16 through which the medium is discharged outside the waste-liquid holding means 14, and serves as a medium-discharging means. The waste-liquid supply port 15 is installed in a top surface of the waste-liquid holding means 14, and the medium that has been supplied to the waste-liquid supply port 15 is made to drip in a space in the waste-liquid holding means and is discharged through the waste-liquid discharging port 16. In this way, by making the medium drip in the space in the waste-liquid holding means 14, it is possible to prevent a reverse flow of the medium, and thus, it is possible to prevent the interior of the cell-culturing container 11 from becoming polluted (the occurrence of contamination).

Fig. 1B shows an example of a cell-culturing system provided with four sets of the medium-supplying means 6 and the cell-culturing containers 11.

Next, the dripping-speed adjusting means 7 will be described.

The dripping-speed adjusting means 7 is disposed in the tubular member (tube or the like) that connects the discharging port 5 of the temporary holding means 4 and the supply port 9 of the dripping container 8, and suppresses the flow speed by restricting the flow volume of a solution by decreasing the cross-sectional area of the internal cavity in the tubular member by deforming the tubular member by applying an external force thereto. In contrast, when the external force is released, the tubular member returns to the original state due to the elastic force in the tubular member, and thus, it is possible to increase the flow speed. In this way, the dripping-speed adjusting means 7 adjusts the flow speed of the solution flowing inside the tubular member by means of the magnitude of the external force applied to the tubular member. Figs. 2A to 2D show examples of manners in which an external force is applied to the tubular member by means of the dripping-speed adjusting means 7. Fig. 2A shows an example in which a tubular member 20 is squeezed by two plate-like members 21; Fig. 2B shows an example in which the tubular member 20 that is made to pass through a through-hole 23 is squeezed by a plurality of spherical (or columnar) members 22; Fig. 2C shows an example in which the tubular member 20 that is made to pass through a through-hole 25 is squeezed by a shutter-like member 24; and Fig. 2D shows an example in which the inner diameter of a through-hole 26 through which the tubular member 20 is made to pass is decreased, thus deforming the tubular member. Mechanisms other than these may be employed so long as the tubular member can be deformed by applying an external force thereto.

In addition, a liquid-feeding pump, such as a peristaltic pump or the like, may be employed as the dripping-speed adjusting means 7.

Next, example procedures for replacing the medium by using the medium replacing system 100 according to this embodiment will be described.

A user of this system first sets the system to a state in which the dripping speed is zero, that is, a state in which dripping is stopped, by using the dripping-speed adjusting means 7. The medium in the medium-preserving means 3 and the temporary holding means 4 is replenished, and the temperature of the medium in the temporary holding means 4 is made to reach the temperature in the incubator. The cell-culturing container 11 containing the medium and cells is prepared, and, in the incubator, the supply port 12 of the cell-culturing container 11 is connected to the discharging port 10 of the dripping container 8 via the tubular member, and the discharging port 13 of the culturing container 11 is connected to the waste-liquid supply port 15 of the waste-liquid holding means 14 via the tubular member.

When the medium needs to be replaced, the user first adjusts the dripping-speed adjusting means 7, thus setting the dripping speed to an appropriate speed. Once the state in which the dripping speed is zero is canceled by using the dripping-speed adjusting means 7, the medium starts to drip into the dripping container 8 due to the action of gravity. The dripping speed may be adjusted so as to be set at a preset speed, or the dripping speed may be appropriately adjusted by the user while visually checking the dripping speed.

Once dripping has started, the medium is supplied to the cell-culturing container 11 via the dripping container 8, and the medium exceeding a defined amount in the cell-culturing container 11 is discharged from the discharging port 13 of the cell-culturing container 11, thus being discharged outside the incubator via the waste-liquid holding means 14. By doing so, the old medium in the cell-culturing container 11 is replaced with new medium, and thus, it is possible to decrease the speed at which the medium is deteriorated.

### {Second Embodiment}

As shown in Figs. 3A and 3B, a cell-culturing system 200 according to this embodiment differs from that of the first embodiment in that the dripping container 8 is not provided and that the supply port 12 of the cell-culturing container 11 is disposed in a top surface of the cell-culturing container 11. Other aspects are the same as those of the first embodiment.

In this case, because the supply port 12 of the cell-culturing container 11 is positioned in the top surface of the cell-culturing container 11, the supplied medium is made to drip in the space in the cell-culturing container 11 toward the medium surface, thus functioning in the same manner as does the dripping container 8. Therefore, it is possible to prevent a reverse flow of the medium in this embodiment also, and thus, it is possible to prevent the medium in the temporary holding means 4 and the medium-preserving means 3 from becoming polluted (the occurrence of contamination).

In addition because the dripping container 8 is not installed, it is possible to make the system compact.

Note that, because the dripping container 8 is not provided, the discharging port 5 of the temporary holding means 4 and the supply port 12 of the cell-culturing container 11 are directly connected via a tubular member, and the dripping-speed adjusting means 7 is installed in that tubular member.

Figs. 4A and 4B show a cell-culturing system 300 as an example of a modification of this embodiment.

With this modification, the dripping container 8 is employed in the second embodiment, as with the first embodiment. By doing so, the medium is made to drip in the dripping container 8 and the cell-culturing container 11, and thus, it is possible to further reduce the risk of polluting the medium in the temporary holding means 4 and the medium-preserving means 3 (the occurrence of contamination) due to a reverse flow of the medium.

### {Third Embodiment}

As shown in Figs. 5A and 5B, a cell-culturing system 400 according to this embodiment differs from those of the above-described individual embodiments in that a pressurizing means 60 that applies pressure to the medium in the medium-preserving means 3 is provided. Other aspects are the same as those of the above-described individual embodiments. Although Figs. 5A and 5B show an example that corresponds to the first embodiment, the example is also applicable to other embodiments.

Examples of the pressurizing means 60 include a means that feeds gas into the medium-preserving means 3 by using a pump. By doing so, the pressure in an air layer in the medium-preserving means 3 is increased, and thus, it is possible to apply external pressure to the medium. It is preferable that the gas to be fed into the medium-preserving means 3 be sterilized gas. As shown in Fig. 6A, the gas (or liquid) is fed into a sac-like member 61, and thus, pressure can be applied to the medium by using the bag whose volume has been increased; or, as shown in Fig. 6B, an air layer 63 and the medium are separated by using a movable isolating member 62, while ensuring satisfactory airtightness, and thus, pressure can also be applied to the medium by using the isolating member 62 by feeding the gas (or liquid) into the air layer 63. By doing so, it is possible to reduce the risk of polluting the medium. Note that, instead of feeding the gas (or liquid) in the form in which the isolating member is employed, as shown in Fig. 6B, a certain amount of pressure may be applied to an isolating member 64 by placing a weight 65 having a predetermined mass on top of the isolating member 64, as shown in Fig. 6C. A medium-holding means and a bulkhead member may have structures like a cylinder and a pusher (plunger) of a syringe, respectively, and pressure may be applied to the medium inside the medium-holding means by mechanically moving the bulkhead member, which corresponds to the pusher (plunger). In this case, the movement of the bulkhead member may be controlled by means of a control portion via wired or wireless communication.

In addition, it is permissible to employ a form in which the medium-holding means has a columnar structure, the bulkhead member has a discoid structure that may be fitted at an inner wall of that columnar structure while maintaining airtightness, and threaded structures that engage with each other may be provided at the inner wall of the columnar structure and the outer circumference of the discoid structure. In this case, when the bulkhead member is attached to the interior of the medium-holding means and rotated in the discoid circumferential direction, the bulkhead member is moved in the height direction of the columnar structure, and thus, it is possible to apply pressure to (or to remove pressure from) the medium in the interior of the medium-holding means. The bulkhead member may be mechanically rotated, and, by doing so, the rotation of the bulkhead member may be performed by means of the control portion via wired or wireless communication.

Here, it is preferable that the pressurizing means 60 be controlled so that the pressure in the medium-preserving means 3 does not reach or exceed a certain level.

Note that, in this embodiment, the medium-preserving means 3 does not necessarily need to be installed at a position that is higher than the temporary holding means 4. In addition, the temporary holding means 4 does not necessarily need to be installed at a position that is higher than the medium-supplying means 6.

### {Fourth Embodiment}

As shown in Figs. 7A and 7B, a cell-culturing system 500 according to this embodiment differs from those of the above-described individual embodiments in that a negative-pressure supplying means 71 that applies negative pressure at the discharging port 13 of the cell-culturing container 11 is provided. Other aspects are the same as those of the above-described individual embodiments. Although Figs. 7A and 7B show an example that corresponds to the first embodiment, the example is also applicable to other embodiments.

Examples of the negative-pressure supplying means 71 include a means provided with a pump 81 and a waste-liquid container 82, as shown in Fig. 8. Negative pressure is generated in the waste-liquid holding means 14 by connecting a suction port 83 of the negative-pressure supplying means 71 to the waste-liquid discharging port 16 of the waste-liquid holding means 14, thus generating negative pressure at the discharging port 13 of the culturing container 11, and, by doing so, the medium can be sucked out of the culturing container 11.

A liquid-feeding pump, such as a peristaltic pump or the like, may be used as the pump of the negative-pressure supplying means. In this case, the liquid-feeding pump may be installed in a tubular member of the suction port 83.

### {Fifth Embodiment}

As shown in Figs. 9A and 9B, with a cell-culturing system 600 according to this embodiment, the dripping-speed adjusting means 7 in the above-described individual embodiments can be remotely operated by means of a control portion 19 installed outside the incubator via wireless or wired communication. Other aspects are the same as those of the above-described individual embodiments. Although Figs. 9A and 9B show an example that corresponds to the first embodiment, the example is also applicable to other embodiments.

In this embodiment, the dripping-speed adjusting means 7 is configured so as to allow information exchange with the control portion 19 installed outside the incubator via wireless or wired communication, and thus, the flow speed of a solution, such as a medium or the like, that flows inside the tubular member (tube or the like) can be remotely controlled.

When the medium needs to be replaced, the user remotely adjusts the dripping-speed adjusting means 7 by means of the control portion 19, thus setting the dripping speed to an appropriate speed. The dripping speed may be adjusted so as to be set at a preset speed, or the dripping speed may be adjusted so as to achieve an appropriate dripping speed while monitoring the dripping speed by using a monitoring system (not shown).

With this embodiment, the user can remotely start medium replacement or can remotely change the dripping speed at an arbitrary timing during cell culturing. For example, by using a system (not shown) with which the state of cells can be remotely monitored together with the system of this embodiment, it is possible to remotely start medium replacement or remotely change the dripping speed at an arbitrary timing during cell culturing in accordance with the state of the cells. By doing so, because the user can perform work without having to enter the workspace, it is possible to reduce the time and effort, as well as costs, involved in changing into disposable work clothes or the like, and it is also possible to reduce the risk of contaminating the cell-culturing system with bacteria or the like.

The control portion 19 of this embodiment may be configured so as to allow information exchange with the temperature-controlling means 2, the pressurizing means 60, and the negative-pressure supplying means 71 of the above-described individual embodiments via wireless or wired communication and so as to allow remote control thereof. By doing so, it is possible to increase the efficiency of work remotely performed by the user.

In the above-described individual embodiments, it is possible to use a cell-culturing container 91 shown in Figs. 10A and 10B. The cell-culturing container 91 has a supply port 93 and a discharging port 94 in a lid portion 92 of the container.

The supply port 93 is connected to the medium-supplying means 6 via a tubular member, and a supplying-pipe portion 95 extends into the interior of the cell-culturing container from the supply port 93. The medium that has been supplied from the medium-supplying means 6 passes through the supply port 93 and enters the supplying-pipe portion 95. As shown in Fig. 10A, the distal end of the supplying-pipe portion 95 is disposed above the surface of the medium, and the medium that has exited the supplying-pipe portion 95 is made to drip in the cell-culturing container 91. Note that, in a form in which the dripping container 8 is employed, the distal end of the supplying-pipe portion 95 does not necessarily need to be disposed above the surface of the medium, as in the example shown in Fig. 10B.

The discharging port 94 is connected to the waste-liquid holding means 14 via a tubular member, and a discharging-pipe portion 96 extends toward the interior of the cell-culturing container from the discharging port 94. The distal end of the discharging-pipe portion 96 is configured so as to be disposed at a position that is at a predetermined height from the bottom surface of the interior of the cell-culturing container, and the medium in the cell-culturing container is discharged outside the cell-culturing container when the medium reaches the height of the distal end of the discharging-pipe portion 96. The amount of medium to be held in the cell-culturing container can be adjusted by the height at which the distal end of the discharging-pipe portion 96 is disposed.

At this time, the replacement efficiency of the medium is increased by increasing the distance between the distal end of the supplying-pipe portion 95 and the distal end of the discharging-pipe portion 96 as much as possible.

An Example of a mechanism for positioning the supplying-pipe portion 95 and the discharging-pipe portion 96 in the lid portion 92 of the cell-culturing container 91 will now be described by using Figs. 11A and 11B.

The lid portion 92 is formed of a lid main body 112 to be attached to a neck portion 97 of a cell-culturing container main body, a discoid member 111 in which the supply port 93 and the discharging port 94 are provided, and a ring-like member 113 in which a thread that engages the ring-like member 113 and the inner side of the lid main body 112 is formed at the outer circumference thereof. Threads that engage with each other are formed at the inner circumference of the lid main body 112 and the outer circumference of the neck portion 97 of the cell-culturing container main body, and the lid main body 112 can be secured to the neck portion 97 by rotating the lid main body 112. The discoid member 111 is a discoid having a diameter that allows independent rotation thereof in the circumferential direction inside the lid main body 112, and thus, it is possible to adjust, by rotating the discoid member 111, the positions of the supplying-pipe portion 95 and the discharging-pipe portion 96 so as to be appropriately placed in the cell-culturing container. In order to place the discoid member 111 so as to allow independent rotation thereof with respect to the lid main body 112, for example, as shown in Figs. 11A and 11B, the discoid member 111 is fitted inside the lid main body 112 first, and the discoid member 111 is attached so as to be rotatable with respect to the lid main body 112 by screwing the ring-like member 113 into the lid main body 112. When the lid main body 112 is screwed into the neck portion 97 in this state, because the discoid member 111 is squeezed between the neck portion 97 and the ring-like member 113, the lid main body 112 is screwed into the neck portion 97 while positioning the supplying-pipe portion 95 and the discharging-pipe portion 96, and thus, the discoid member is squeezed. Note that, during cell culturing, the interior of the cell-culturing container is used as an open system by loosening the screwed state between the lid main body 112 and the neck portion 97 or by loosening the screwed state between the ring-like member 113 and the lid main body 112.

Another example of the mechanism for positioning the supplying-pipe portion 95 and the discharging-pipe portion 96 in the lid portion 92 of the cell-culturing container 91 will now be described.

A protrusion is provided at an inner side of the lid portion 92, whereas the neck portion 97 of the container main body is provided with a depression to which the protrusion of the neck portion 97 is fitted. When the lid portion 92 is fitted to the neck portion 97, the protrusion and the depression should be positioned so that the supplying-pipe portion 95 and the discharging-pipe portion 96 are disposed at appropriate positions in the cell-culturing container by engaging the protrusion with the depression.

Fig. 12 shows a more specific example of a structure in the vicinity of the lid portion. The neck portion 97 is provided with two ring-like members 102 with a space therebetween in the circumferential direction, and a depression 103 is provided between the two ring-like members 102. The protrusion 101 at the inner side of the lid portion 92 is configured so as to be fitted between the two ring-like members 102 when the lid portion 92 is fitted to the neck portion 97, and, at this time, the lid portion 92 is in a state in which the lid portion 92 can be rotated in the circumferential direction of the neck portion 97. The configuration here is such that the supplying-pipe portion 95 and the discharging-pipe portion 96 can be disposed at appropriate positions in the cell-culturing container when the protrusion 101 and the depression 103 are engaged with each other.

Although a form in which the protrusion of the lid portion and the depression in the neck portion are engaged with each other is shown in Fig. 12, both the lid portion and the neck portion may have protrusions, and the configuration thereof may be such that the supplying-pipe portion 95 and the discharging-pipe portion 96 may be disposed at appropriate positions in the cell-culturing container at positions at which these protrusion abut each other.

In the above-described individual embodiments, although flask-like culturing containers have been shown in the drawings as the culturing containers to be used, petri-dish-like culturing containers or the like may be used, as shown in Figs. 13A to 13D. Note that, in Figs. 13C and D, the same reference signs are assigned to components corresponding to the cell-culturing container of Fig. 10.

In addition, a sac-like cell-culturing bag provided with a supply port that supplies a solution and a discharging port that discharges the solution may be employed.

Furthermore, as shown in Figs. 14A to 14D, a cell-culturing container having a partition with which a flow channel is formed may be employed. By using this container, the medium replacement efficiency is enhanced by installing a supply port 141 and a discharging port 142 at positions away from each other along the flow channel.

In the above-described individual embodiments, although examples in which the waste liquid discharged from the discharging port of the culturing container is discharged via the waste-liquid holding means have been described, for example, as shown in Figs. 15A and 15B, the waste liquid may be directly discharged from the discharging port of the cell-culturing container without providing the waste-liquid holding means. Although Figs. 15A and 15B show an example that corresponds to the first embodiment, the example is also applicable to other embodiments.

In this case, in the third embodiment, the suction ports of the negative-pressure supplying means may be connected to the individual discharging ports of the culturing containers.

In the above-described individual embodiments, although forms in which the medium-preserving means is employed have been described, for example, as shown in Figs. 16A and 16B, the medium-preserving means may be omitted. In this case, the medium in the temporary holding means may directly be replenished, and it is preferable that the temporary holding means be provided with a supply port for this purpose. The temporary holding means may be configured for single use without providing the supply port. Although Figs. 16A and 16B show an example that corresponds to the first embodiment, the example is also applicable to other embodiments. The third embodiment may take a form in which the pressurizing means 60 applies pressure to the medium in the temporary holding means instead of the medium-preserving means.

The above-described individual embodiments have been described in terms of forms in which the medium in the medium-preserving means 3 is supplied to the temporary holding means 4 via the tubular member (tube or the like) by the action of gravity because the medium-preserving means 3 is connected to the temporary holding means 4 via the tubular member (tube or the like) and the medium-preserving means 3 is installed at a position that is higher than the temporary holding means 4 in the direction in which gravity acts. For example, as shown in Figs. 17A and 17B, the medium may be supplied to the temporary holding means from the medium-preserving means by installing a liquid-feeding pump 121, such as a peristaltic pump or the like, in the tubular member (tube or the like) that connects the medium-preserving means and the temporary holding means. In this case, the medium-holding means 3 does not necessarily need to be installed at a position that is higher than the temporary holding means 4 in the direction in which gravity acts, and thus, the degree of freedom with respect to the sites at which the medium-holding means 3 is installed is increased. In this case, the liquid-feeding pump 121, such as a peristaltic pump or the like, may be remotely operated by means of a controlling means. Remote operation may be performed via wired or wireless communication. Although Figs. 17A and 17B show an example that corresponds to the first embodiment, the example is also applicable to other embodiments.

Although the above-described individual embodiments have been described in terms of forms in which the temporary holding means is employed, in the case in which the temperature of the medium is raised to an appropriate temperature for cell culturing before the medium is supplied to the cell-culturing container, the temporary holding means does not necessarily need to be employed. For example, in the case in which the dripping speed is sufficiently low or in the case in which the dripping container has a sufficiently large capacity, it is possible not to employ the temporary holding means because the temperature of the medium reaches an appropriate temperature for cell culturing before the medium is supplied to the cell-culturing container. In this case, the medium-preserving means and the medium-supplying means may be directly connected by using the tubular member (tube or the like). For example, as in Fig. 21A, the medium-preserving means may be provided with a plurality of discharging ports, and the plurality of discharging ports may individually be connected to different medium-supplying means; or, for example, as in Fig. 21B, a tubular member leading to the discharging port of the medium-preserving means may be split into a plurality of tubular members at an intermediate position thereof, and the plurality of tubular members may individually lead to different medium-supplying means. The set temperature of the solution, such as a medium or the like, in the medium-preserving means may be an appropriate temperature for preservation thereof (for example, 4 °C) or an appropriate temperature for cell culturing (for example, 37 °C), and it is possible to appropriately set the temperature depending on the culturing conditions.

In addition, for example, as shown in Figs. 22A and 22B, the medium may be supplied to the medium-supplying means from the medium-preserving means by installing a liquid-feeding pump, such as a peristaltic pump or the like, in the tubular member (tube or the like) that connects the medium-preserving means and the medium-supplying means. By doing so, the medium-holding means does not necessarily need to be installed at a position that is higher than the medium-supplying means in the direction in which gravity acts, and thus, the degree of freedom with respect to the sites at which the medium-preserving means is installed is increased. In this case, because the liquid-feeding pump serves as the dripping-speed adjusting means of the medium-supplying means, the dripping-speed adjusting means may be omitted. The liquid-feeding pump, such as a peristaltic pump or the like, may be installed inside or outside the incubator, and may be remotely operated by means of the control means via wired or wireless communication. In addition, as shown in Figs. 22A and 22B, the supply port of the cell-culturing container may be installed at the top surface of the container, and the solution, such as a medium or the like, may be made to drip therefrom without employing the dripping container.

Although Figs. 21A and 21B show an example that corresponds to the first embodiment, the example is also applicable to other embodiments. In addition, although Figs. 22A and 22B show an example that corresponds to the first embodiment, the example is also applicable to other embodiments.

Although the above-described individual embodiments have forms in which one each of the medium-holding means and the temporary holding means are provided, for example, as shown in Figs. 18A and 18B, more than one each of the medium-holding means and the temporary holding means may be provided. In this case, it is possible to supply multiple types of media to the cell-culturing container. Regarding the medium-supplying means connected to the temporary holding means, a form in which the medium-supplying means are individually provided for the plurality of temporary holding means, for example, as shown in Figs. 18A and 18B, may be employed; or a form in which the plurality of temporary holding means are connected to a single medium-supplying means, for example, as shown in Figs. 19A and 19B, may be employed. Although Figs. 18A and 18B and Figs. 19A and 19B show examples that correspond to the first embodiment, the examples are also applicable to other embodiments.

Although the above-described individual embodiments have been described in terms of forms in which a single medium-supplying means leads to a single cell-culturing container, a form in which a single medium-supplying means leads to a plurality of cell-culturing containers may be employed. For example, as shown in Fig. 20A, the dripping container may be provided with a plurality of discharging ports, and the plurality of the discharging ports may individually lead to supply ports of different cell-culturing containers; or, for example, as shown in Fig. 20B, a tubular member leading to the discharging port of the dripping container may be split into a plurality of tubular members at an intermediate position thereof, and the plurality of tubular members may individually lead to supply ports of different cell-culturing containers. Although Figs. 20A and 20B show examples that correspond to the first embodiment, the examples are also applicable to other embodiments.

In the above-described individual embodiments, a liquid-feeding pump, such as a peristaltic pump or the like, may be used as the dripping-speed adjusting means. The liquid-feeding pump may be remotely controlled by means of the control portion via wired or wireless communication.

In the above-described individual embodiments, a medium-temperature monitoring means that monitors the medium temperature may be installed in the temporary holding means. In this case, the medium-temperature monitoring means may be configured so as to remotely transmit the monitored medium-temperature information to the control portion. By doing so, it is possible to prevent the medium at a temperature that is not appropriate for cell culturing from being accidentally supplied.

The cell-culturing systems of the present invention can be applied to, as a matter of course, culturing of adhesive cells, as well as to culturing of non-adhesive cells. This is because it is possible to reduce the risk of non-adhesive cells flowing out of the discharging port of the cell-culturing container by making it possible to set the medium-replacement speed to be low and by disposing the discharging port of the cell-culturing container on an upper side in the height direction.

In addition, by making the amount of medium held in the cell-culturing container as small as possible, it is possible to enhance the medium replacement efficiency. The amount of medium held in the cell-culturing container may be optimized depending on the type of cells to be cultured.

In the above-described individual embodiments, the control portion may be configured so as to allow information exchange with the temperature-controlling means, the pressurizing means, the negative-pressure supplying means, and the liquid-feeding pump of the above-described individual embodiments via wireless or wired communication and so as to allow remote control thereof. By doing so, it is possible to increase the efficiency of the work remotely performed by the user.

In the above-described individual embodiments, with respect to the form in which the user remotely performs the medium replacement by means of the control portion, the control portion may remotely perform the medium replacement on the basis of a schedule (program) set by the user in advance.

Although the above-described individual embodiments have been described in terms of forms in which the temporary holding means is installed inside the incubator, the temporary holding means may be installed outside the incubator. In that case, it is preferable that a temperature-controlling means that maintains the temperature of the solution in the temporary holding means at an appropriate temperature for cell culturing (for example, 37 °C) be installed.

In addition, although the above-described individual embodiments have been described in terms of forms in which the waste-liquid holding means is installed inside the incubator, for example, as shown in Figs. 22A and 22B, the waste-liquid holding means may be installed outside the incubator.

In the above-described individual embodiments, it is possible to use a cell-culturing container shown in Fig. 23A. The cell-culturing container is provided with a rack-like structure formed of a plate-like member 130 inside the container. The plate-like member forms the rack-like structure by being secured inside the container, and is provided with a solution stopper 131 at a top-surface portion thereof so that a predetermined amount of solution can be held. The plate-like member 130 is secured inside the container such that top and bottom spaces partitioned by the plate-like member 130 communicate with each other through an opening 132. A supply port 133 is provided at the top surface of the container, and a solution that is supplied therefrom is supplied to the rack portion formed by the plate-like member 130. The predetermined amount of solution is held on the plate-like member 130 by the solution stopper 131, and the solution exceeding the predetermined amount flows over the solution stopper 131 and is made to drip to the lower space from the opening 132. A discharging port 134 is provided in a side surface of the container at a position at a predetermined height from the bottom surface, and the solution is discharged therefrom when the solution held in the lower space reaches the height at which the discharging port 134 is located. It suffices that the supply port 133 be located at a position from which the solution can be supplied to the rack portion formed by the plate-like member 130, and the supply port 133 may be provided at the side surface of the container. In addition, separately from the supply port 133, a supply port 133' may additionally be provided at a position from which the solution can directly be supplied to the lower space, as shown in Fig. 23B. Although an example in which one plate-like member forms one rack portion is shown in the figure, two or more plate-like members may form two or more rack portions. With such a cell-culturing container, it is possible to increase the area of culturing space for culturing cells, and thus, it is possible to efficiently multiply the cells.

In the above-described individual embodiments, a waste-liquid-speed adjusting means may be provided in a tubular member that connects the discharging port of the cell-culturing container and the waste-liquid supply port of the waste-liquid holding means. The waste-liquid-speed adjusting means is capable of controlling the flow speed of the waste liquid that flows inside the tubular member, and the structure thereof is the same as that of the dripping-speed adjusting means shown in Figs. 2A to 2D. A liquid-feeding pump, such as a peristaltic pump or the like, may be employed as the waste-liquid-speed adjusting means. The waste-liquid-speed adjusting means may be configured so as to allow remote operation thereof by means of a control portion installed outside the incubator via wireless or wired communication.

Examples of the control portions of the present invention include a PC, and the PC can perform control executed by the control portions in the above-described individual embodiments.

In the present invention, the medium-preserving means and the temporary medium-holding means are means capable of holding the medium or other solution (for example, cleaning liquid or the like), each of which serves as a solution-holding means. In other words, with a form in which only the medium-preserving means or the temporary medium-holding means is provided, each of them serves as the solution-holding means, and in a form in which the medium-preserving means and the temporary medium-holding means are provided, both means integrally serve as the solution-holding means.

With the present invention, it is possible to provide a cell-culturing system including: a medium-preserving means that maintains a medium for culturing cells at an appropriate temperature for preserving the medium; a temporary medium-holding means that is connected to the medium-preserving means and that maintains the medium supplied thereto from the medium-preserving means at an appropriate temperature for cell culturing; a medium-supplying means that is connected to the temporary medium-holding means and that supplies the medium supplied thereto from the temporary medium-holding means to the cell-culturing container; and a cell-culturing container that is connected to the medium-supplying means and that cultures cells in the medium supplied thereto from the medium-supplying means, the cell-culturing system being characterized in that the medium-supplying means supplies the medium to the cell-culturing container via a medium dripping step.

With the present invention, it is possible to provide a cell-culturing system including: a medium-preserving means that maintains a medium for culturing cells at an appropriate temperature for preserving the medium; a temporary medium-holding means that is connected to the medium-preserving means and that maintains the medium supplied thereto from the medium-preserving means at an appropriate temperature for cell culturing; a medium-supplying means that is connected to the temporary medium-holding means and that supplies the medium supplied thereto from the temporary medium-holding means to the cell-culturing container; a cell-culturing container that is connected to the medium-supplying means and that cultures cells in the medium supplied thereto from the medium-supplying means; and a medium-discharging means that is connected to the cell-culturing container and that discharges the medium supplied thereto from the cell-culturing container, the cell-culturing system being characterized in that the medium-discharging means discharges the medium via a medium dripping step.

With the present invention, it is possible to provide a cell-culturing system including: a solution-holding means that holds a medium; and a medium-supplying means that is connected to the solution-holding means and that supplies the medium supplied thereto from the solution-holding means to a cell-culturing container, the cell-culturing system being characterized in that the medium-supplying means supplies the medium to the cell-culturing container via a medium dripping step.

With the present invention, it is possible to provide a cell-culturing system including: a solution-holding means that holds a medium; a medium-supplying means that is connected to the solution-holding means and that supplies the medium supplied thereto from the solution-holding means to a cell-culturing container; and a medium-discharging means that is connected to the cell-culturing container and that discharges the medium supplied thereto from the cell-culturing container, the cell-culturing system being characterized in that the medium-discharging means discharges the medium via a medium dripping step.

Cell-culturing systems according to additional embodiments of the present invention will be described below with reference to the drawings.

### {Sixth Embodiment}

A cell-culturing system 1800 according to this embodiment is a system having a configuration shown in Fig. 24, and is a system with which a medium in a cell-culturing container (culture vessel) installed in an incubator is replaced.

Solution-holding means 3 (a first solution-holding means 3a and a second solution-holding means 3b) are installed inside the incubator 1, and are means that preserve, in the interior thereof, a medium (cell-culturing liquid) or a liquid such as a cleaning liquid or the like. Fig. 24 shows an example in which the first solution-holding means 3a holds a medium (cell-culturing liquid) and a second solution-holding means 3b holds cleaning liquid. The solution held in the solution-holding means 3 is warmed so as to reach the temperature in the incubator, which is an appropriate temperature for cell culturing (for example, 37 °C).

The solution that has been warmed to the appropriate temperature for cell culturing (for example, 37 °C) by using the solution-holding means 3 is supplied to the cell-culturing container 11 in the incubator 1 via solution-supplying means 6 (a first solution-supplying means 6a and a second solution-supplying means 6b).

The solution-supplying means 6 are provided with dripping-speed adjusting means 7 (a first dripping-speed adjusting means 7a and a second dripping-speed adjusting means 7b) and dripping containers 8 (a first dripping container 8a and a second dripping container 8b). The dripping-speed adjusting means 7 are installed in tubular members (tubes or the like) that connect the solution-holding means 3 and the dripping containers 8, and are capable of controlling the flow speed of the solution that flows inside the tubular members. The first dripping-speed adjusting means 7a is installed in a tubular member that connects the first solution-holding means 3a and the first dripping container 8a, and the second dripping-speed adjusting means 7b is installed in a tubular member that connects the second solution-holding means 3b and the second dripping container 8b.

The dripping-speed adjusting means 7 is configured so as to allow remote operation thereof by means of the control portion 19 installed outside the incubator via wireless or wired communication, and thus, it is possible to remotely stop the flow of the solution, such as a medium or the like, flowing inside the tubular members (tube or the like) (achieve a state in which the flow speed thereof is zero) or to remotely control the flow speed thereof.

When the medium needs to be replaced, the user remotely adjusts the dripping-speed adjusting means 7 by means of the control portion 19, thus setting the dripping speed to an appropriate speed. The dripping speed may be adjusted so as to be set at a preset speed, or the dripping speed may be adjusted so as to achieve an appropriate dripping speed while monitoring the dripping speed by using a monitoring system (not shown).

The dripping containers 8 are containers having supply ports 9 that are connected to the tubular members leading thereto from the solution-holding means 3 and discharging ports 10 that are connected to tubular members (tubes or the like) leading to the cell-culturing container 11. The supply ports 9 are disposed at upper portions in the direction in which gravity acts, and the discharging ports 10 are disposed at lower portions than the supply ports 9 in the direction in which gravity acts; and thus, the solution supplied to the supply ports 9 is made to drip in the spaces in the dripping containers 8, is discharged from the discharging ports 10, and is supplied to the cell-culturing container 11 via the tubular members.

As has been described above, by making the solution drip in the spaces in the dripping containers 8, it is possible to prevent a reverse flow of the solution, and thus, it is possible to prevent the solution in the solution-holding means 3 from becoming polluted (the occurrence of contamination).

The cell-culturing container 11 has supply ports 12 (a first supply port 12a and a second supply port 12b) that are connected to the tubular members leading thereto from the dripping containers 8 and a discharging port 13 from which the solution is discharged outside the cell-culturing container. The first supply port 12a is connected to the discharging port 10 of the first dripping container 8a via the tubular member, and the second supply port 12b is connected to the discharging port 10 of the second dripping container 8b via the tubular member.

The solution that has passed through the supply ports 12 is made to drip in the space in the culturing container, thus being supplied to the culturing container. At this time, the solution may be supplied without making the solution drip by installing the tubular members (tubes or the like) so as to reach into the interior of the culturing container from the supply ports 12. In addition, in the case in which the solution is made to drip, it is desirable that positions at which the supply ports 12 are installed be in the lid of the cell-culturing container as shown in Fig. 24; however, for example, the supply ports may be installed at the side surface of the cell-culturing container.

On the other hand, for example, as shown in Fig. 24, the discharging port 13 is installed at the lid of the cell-culturing container and has a structure in which the tubular member (tube or the like) extends into the interior of the cell-culturing container. This tubular member has a length that substantially reaches the bottom surface of the cell-culturing container, and is capable of discharging the solution at a bottom-surface portion of the cell-culturing container. By doing so, it is possible to discharge nearly the entire amount of solution in the culturing container. In addition, the tubular member that extends into the interior of the cell-culturing container from the discharging port 13 may have a hole 28 at a side surface thereof, as shown in Fig. 25, and the solution may be discharged through this hole 28. In this case, it is also possible to discharge the solution by disposing the distal end of the tubular member along the bottom surface of the culturing container.

The position at which the discharging port 13 is installed is arbitrary so long as the solution at the bottom-surface portion of the cell-culturing container can be discharged; for example, the discharging port 13 may be installed in a side surface or a bottom surface of the cell-culturing container. In this case, the tubular member that extends into the interior of the cell-culturing container may be omitted.

The discharging port 13 of the cell-culturing container 11 is connected to the solution-discharging means via the tubular member (tube or the like). The solution-discharging means is provided with the waste-liquid holding means 14 and the negative-pressure supplying means 71. The waste-liquid holding means 14 is connected to the discharging port 13 of the cell-culturing container 11 via the tubular member and has the waste-liquid supply port 15 that is connected to that tubular member and the waste-liquid discharging port 16 from which the waste liquid is discharged outside the waste-liquid holding means 14. The position at which the waste-liquid holding means 14 is installed may be inside or outside the incubator. The waste-liquid supply port 15 is installed at the top surface of the waste-liquid holding means 14; and the waste liquid supplied to the waste-liquid supply port 15 is made to drip in a space in the waste-liquid holding means and is discharged through the waste-liquid discharging port 16. As has been described above, by making the waste liquid drip in the space in the waste-liquid holding means 14, it is possible to prevent a reverse flow of the waste liquid, an thus, it is possible to prevent the interior of the cell-culturing container 11 from becoming polluted (the occurrence of contamination). A tubular member that connects the discharging port 13 of the cell-culturing container 11 and the waste-liquid supply port 15 is provided with the waste-liquid-speed adjusting means 17. The waste-liquid-speed adjusting means 17 is capable of controlling the flow speed of the waste liquid flowing inside the tubular member.

The waste-liquid-speed adjusting means 17 is configured so as to allow remote operation thereof by means of the control portion 19 installed outside the incubator via wireless or wired communication, and thus, it is possible to remotely stop the flow of the waste liquid in the tubular member (tube or the like) (achieve a state in which the flow speed thereof is zero) or to remotely control the flow speed thereof.

When the medium needs to be replaced, the user remotely adjusts the waste-liquid-speed adjusting means 17 by means of the control portion 19, thus setting the discharging speed to an appropriate speed. The discharging speed may be adjusted so as to be set at a preset speed, or the discharging speed may be adjusted so as to achieve an appropriate speed while monitoring the discharging speed by using a monitoring system (not shown). The structure of the waste-liquid-speed adjusting means 17 is the same as that of the dripping-speed adjusting means 7 shown in Figs. 2A to 2D.

The waste-liquid discharging port 16 of the waste-liquid holding means 14 is connected to the negative-pressure supplying means 71, and thus, it is possible to generate negative pressure at the waste-liquid holding means 14.

Examples of the negative-pressure supplying means 71 include a means provided with a pump 31 and a waste-liquid container 32, as shown in Fig. 26. Negative pressure is generated in the waste-liquid holding means 14 by connecting a suction port 33 of the negative-pressure supplying means 71 to the waste-liquid discharging port 16 of the waste-liquid holding means 14, thus generating negative pressure at the discharging port 13 of the culturing container 11, and, by doing so, the medium can be sucked out of the cell-culturing container 11.

In addition, a liquid-feeding pump, such as a peristaltic pump or the like, may be used as the negative-pressure supplying means 71. In this case, the liquid-feeding pump may be installed in a tubular member of the suction port 33.

Next, the dripping-speed adjusting means 7 will be described.

The dripping-speed adjusting means 7 is disposed in the tubular member (tube or the like) that connects the discharging port 5 of the solution-holding means 3 and the supply port 9 of the dripping container 8, and suppresses the flow speed by restricting the flow volume of the solution by decreasing the cross-sectional area of the internal cavity of the tubular member by deforming the tubular member by applying an external force thereto. In contrast, when the external force is released, the tubular member returns to the original state due to the elastic force of the tubular member, and thus, it is possible to increase the flow speed. In this way, the dripping-speed adjusting means 7 adjusts the flow speed of the solution flowing inside the tubular member by means of the magnitude of the external force applied to the tubular member. The manners in which the external force is applied to the tubular member by means of the dripping-speed adjusting means 7 are the same as those of the first embodiment, examples of which are shown in Figs. 2A to 2D.

Note that the structure of the waste-liquid-speed adjusting means is the same as that of the dripping-speed adjusting means 7.

In addition, a liquid-feeding pump, such as a peristaltic pump or the like, may be employed as the dripping-speed adjusting means 7. A liquid-feeding pump, such as a peristaltic pump or the like, may be employed as the waste-liquid-speed adjusting means. The liquid-feeding pump may be remotely operated by means of the control portion 19.

Next, example procedures for replacing a medium by using the medium replacing system 1800 according to this embodiment will be described.

A user of this system first sets the system to a state in which the dripping speed is zero, that is, a state in which dripping is stopped, by using the dripping-speed adjusting means 7. A solution, such as a medium or the like, in the solution-holding means 3 is replenished, and the temperature of the medium or the solution in the solution-holding means 3 is made to reach the temperature in the incubator. The cell-culturing container 11 containing the medium and cells is prepared, and, in the incubator, the supply port 12 of the cell-culturing container 11 is connected to the discharging port 10 of the dripping container 8 via the tubular member, and the discharging port 13 of the cell-culturing container 11 is connected to the waste-liquid supply port 15 of the waste-liquid holding means 14 via the tubular member, respectively.

When the medium needs to be replaced, the user first operates the waste-liquid-speed adjusting means 17 by means of the control portion 19, thus setting the discharging speed to an appropriate speed. Once the state in which the discharging speed is zero is cancelled by using the waste-liquid-speed adjusting means 17, the negative pressure in the waste-liquid holding means 14 is transmitted to the discharging port 13 of the cell-culturing container, and thus, the medium in the cell-culturing container is sucked out into the waste-liquid holding means 14 as the waste liquid. When the medium in the cell-culturing container is eliminated, the state in which the discharging speed is zero is restored by means of the waste-liquid-speed adjusting means 17.

Next, the user operates the second dripping-speed adjusting means 7b by means of the control portion 19, thus setting the dripping speed to an appropriate speed. Once the state in which the dripping speed is zero is cancelled by using the dripping-speed adjusting means 7b, the cleaning liquid in the second solution-holding means 4b starts to drip in the dripping container 8b due to the action of gravity, thus being supplied to the cell-culturing container. After a desired amount of cleaning liquid is supplied to the cell-culturing container, the user operates the second dripping-speed adjusting means 7b by means of the control portion 19, thus restoring the state in which the dripping speed is zero. Furthermore, the waste-liquid-speed adjusting means 17 is operated by means of the control portion 19, thus discharging the cleaning liquid in the culturing container by setting the discharging speed to an appropriate speed.

Next, the user operates the first dripping-speed adjusting means 7a by means of the control portion 19, thus setting the dripping speed to an appropriate speed. Once the state in which the dripping speed is zero is cancelled by using the dripping-speed adjusting means 7a, the medium in the first solution-holding means 4a starts to drip in the dripping container 8a due to the action of gravity, thus being supplied to the cell-culturing container. After a desired amount of medium is supplied to the cell-culturing container, the user operates the first dripping-speed adjusting means 7a by means of the control portion 19, thus restoring the state in which the dripping speed is zero.

Figs. 27A and 27B show a modification of this embodiment.

A cell-culturing system 1900 according to this modification is a form in which each of a first dripping container 41a and a second dripping container 41b include a plurality of discharging ports. The plurality of discharging ports provided in the first dripping container 41a and the second dripping container 41b are individually connected to supply ports 12 (first supply ports 42a and second supply ports 42b) of different cell-culturing containers via tubular members. Although Fig. 27A shows a form in which two cell-culturing containers are employed, it is possible to install as many cell-culturing containers as the number of discharging ports provided in the individual dripping containers.

Instead of providing a plurality of discharging ports in the individual dripping containers, as shown in Fig. 27B, each of the dripping containers may be provided with one discharging port, and the tubular member leading thereto may be split into a plurality of tubular members at an intermediate position thereof and may individually be connected to the supply ports 12 (the first supply ports 42a and the second supply ports 42b) of different cell-culturing containers.

Other aspects are the same as those of the first embodiment.

Fig. 28 shows another modification of this embodiment.

A cell-culturing system 2100 according to this modification is a form in which the first dripping container 8a and the second dripping container 8b are combined into a single dripping container 8c. The dripping container 8c has two supply ports (a first supply port 43a and a second supply port 43b) for supplying a solution to the interior thereof and one discharging port 44 for discharging the solution from the interior thereof. The first supply port 43a and the second supply port 43b are connected to a first solution-holding means 3a and a second solution-holding means 3b, respectively, via tubular members. The discharging port 44 is connected to a supply port 45 of the cell-culturing container via a tubular member. Other aspects are the same as those of the first embodiment.

In this case, a form in which the first dripping container 8c has a plurality of discharging ports, as shown in Fig. 29A, may be employed. The plurality of discharging ports provided in the dripping container 8c are individually connected to the supply ports 45 of different cell-culturing containers via tubular members. Although Fig. 29A shows a form in which two cell-culturing containers are employed, it is possible to install three or more cell-culturing containers.

Instead of providing a plurality of discharging ports in the dripping container, as shown in Fig. 29B, each of the dripping containers may have one discharging port, and the tubular member leading thereto may be split into a plurality of tubular members at an intermediate position thereof and may individually be connected to the supply ports 45 of different cell-culturing containers.

The dripping container may be provided with a single supply port, the tubular members from the first solution-holding means 3a and the second solution-holding means 3b may be joined at an intermediate position and may be connected to that single supply port.

### {Seventh Embodiment}

As shown in Fig. 30, a cell-culturing system 2400 according to this embodiment differs from that of the sixth embodiment in that the dripping container 8 is not provided, and in that the positions at which supply ports 12 (a first supply port 12a and a second supply port 12b) of the cell-culturing container 11 are disposed are restricted to the top surface of the cell-culturing container 11. Other aspects are the same as those of the sixth embodiment.

In this case, because the supply ports 12 of the cell-culturing container 11 are positioned at the top surface of the cell-culturing container 11, the supplied solution is made to drip in a space in the cell-culturing container 11, and thus, the supply ports 12 have the same function as does the dripping container 8. Therefore, in this embodiment also, it is possible to prevent a reverse flow of the medium, and thus, it is possible to prevent the medium in the solution-preserving means 3 from becoming polluted (the occurrence of contamination). In addition, because the dripping container 8 is not installed, it is possible to make the system compact.

Note that, because the dripping container 8 is not provided, the discharging ports 5 of the solution-holding means 3 and the supply ports 12 of the cell-culturing container 11 are directly connected via tubular members, and the dripping-speed adjusting means 7 are installed in these tubular members. In other words, the first solution-holding means 3a and the second solution-holding means 3b are connected to the first supply port 12a and the second supply port 12b of the cell-culturing container, respectively, via the tubular members, and the first dripping-speed adjusting means 7a and the second dripping-speed adjusting means 7b are installed in the respective tubular members.

### {Eighth Embodiment}

As shown in Fig. 31, a cell-culturing system 2500 according to this embodiment differs from those of the above-described individual embodiments in that solution-stocking means 51 (a first solution-stocking means 51a and a second solution-stocking means 51b) that supply the solution to the solution-holding means 3 are provided. Other aspects are the same as those of the above-described individual embodiments. Although Fig. 31 shows an example that corresponds to the sixth embodiment, the example is also applicable to other embodiments.

The solution-stocking means 51 are provided with temperature-controlling means 52 that maintains the solution, such as the medium or the like, held in the interiors thereof at an appropriate temperature for preserving the solution (for example, 4 °C). The sites at which the solution-stocking means 51 are installed are arbitrary so long as the positions thereof are higher than that of the solution-holding means 3 in the direction in which gravity acts, and the solution-stocking means 51 may be installed inside or outside the incubator.

The solution-stocking means 51 (the first solution-stocking means 51a and the second solution-stocking means 51b) are connected to the solution-holding means 3 (the first solution-holding means 3a and the second solution-holding means 3b) via tubular members (tubes or the like). Because the solution-stocking means 51 are installed at positions that are higher than that of the solution-holding means 3 in the direction in which gravity acts, the solution in the solution-stocking means 51 is supplied to the solution-holding means 3 via the tubular members (tubes or the like) by the action of gravity. The medium that has been supplied to the solution-holding means 3 is warmed so as to reach the temperature in the incubator, which is an appropriate temperature for cell culturing (for example, 37 °C).

Although Fig. 31 shows a form in which the solution is moved to the solution-holding means 3 from the solution-stocking means 51 by means of gravity, as shown in Fig. 32, the solution may be moved to the solution-holding means 3 from the solution-stocking means 51 by installing liquid-feeding pumps 53 (a first liquid-feeding pump 53a and a second liquid-feeding pump 53b), such as peristaltic pumps, in the tubular members (tubes or the like) that connect the solution-stocking means 51 and the solution-holding means 3. In this case, the installation sites of the solution-stocking means 51 need not be positions that are higher than the solution-holding means 3 in the direction in which gravity acts.

### {Ninth Embodiment}

As shown in Fig. 33, a cell-culturing system 2700 according to this embodiment differs from those of the above-described individual embodiments in that pressurizing means 60 that apply pressure to the solution in the solution-holding means 3 are provided. Other aspects are the same as those of the above-described individual embodiments. Although Fig. 33 shows an example that corresponds to the sixth embodiment, the example is also applicable to other embodiments.

Examples of the pressurizing means 60 include means that feeds gas into the solution-holding means 3 by using a pump. By doing so, the pressure in air layers in the solution-preserving means 3 is increased, and thus, it is possible to apply external pressure to the solution. It is preferable that the gas to be fed into the solution-holding means 3 be sterilized gas. Pressurizing means that are the same as the examples shown in Figs. 6A to 6C may be employed.

Here, it is preferable that the pressurizing means 60 be controlled so that the pressure in the solution-holding means 3 does not reach or exceed a certain level.

In this embodiment, the solution-holding means 3 do not necessary need to be installed at positions that are higher than the solution-supplying means 6.

Note that, in a form provided with a solution-stocking means, instead of applying pressure to the solution in the solution-holding means by using the pressurizing means 60, as shown in Fig. 34, a form in which pressure is applied to the solution in the solution-stocking means 51 (the first solution-stocking means 51a and the second solution-stocking means 51b) may be employed. In this case, the solution-stocking means 51 do not necessarily need to be installed at positions that are higher than the solution-holding means 3. In addition, the solution-holding means 3 does not necessarily need to be installed at positions that are higher than the solution-supplying means 6.

In the above-described individual embodiments, it is possible to use a cell-culturing container 91 shown in Figs. 38A and 38B. The cell-culturing container 91 has a supply port 93 and a discharging port 94 at a lid portion 92 of the container.

The supply port 93 is connected to the solution-holding means 6 via a tubular member, and a supplying-pipe portion 95 extends into the interior of the cell-culturing container from the supply port 93. The solution supplied from the solution-supplying means 6 passes through the supply port 93 and enters the supplying-pipe portion 95. As shown in Fig. 38A, the distal end of the supplying-pipe portion 95 is disposed at a predetermined height above the bottom surface of the interior of the cell-culturing container, and the solution that has exited the supplying-pipe portion 95 is made to drip in the cell-culturing container 91. Note that in a form in which the dripping container 8 is employed, as in the example shown in Fig. 38B, the distal end of the supplying-pipe portion 95 does not necessarily need to be disposed at the predetermined height above the bottom surface of the interior of the cell-culturing container.

The discharging port 94 is connected to the waste-liquid holding means 14 via a tubular member, and a discharging-pipe portion 96 extends into the interior of the cell-culturing container from the discharging port 94. The distal end of the discharging-pipe portion 96 is configured so as to be disposed at a position that reaches the bottom surface of the interior of the culturing container, and thus, it is possible to discharge nearly the entire amount of solution in the cell-culturing container.

The discharging-pipe portion 96 may have the hole 28 at a side surface thereof, as shown in Fig. 25, and the solution may be discharged through this hole 28. In this case, it is also possible to discharge the solution by disposing the distal end of the discharging-pipe portion 96 along the bottom surface of the culturing container.

The position at which the discharging port 94 is installed is arbitrary so long as nearly the entire amount of solution in the cell-culturing container can be discharged; for example, the discharging port 94 may be installed in a side surface or a bottom surface of the cell-culturing container. In this case, the discharging-pipe portion 96 may be omitted.

With regard to an example of a mechanism for positioning the supplying-pipe portion 95 and the discharging-pipe portion 96 of the lid portion 92 of the cell-culturing container 91, it is possible to employ the same mechanism as the example shown in Figs. 11A and Fig. 11B.

Fig. 12 shows a more specific example of a structure in the vicinity of the lid portion.

In the above-described individual embodiments, although examples in which the waste liquid discharged from the discharging port of the cell-culturing container is discharged via the waste-liquid holding means have been described, the waste liquid may be directly discharged from the discharging port of the cell-culturing container without providing the waste-liquid holding means. For example, as shown in Fig. 35, the discharging port of the cell-culturing container may be connected to the negative-pressure supplying means 71 via a tubular member. Although Fig. 35 shows an example that corresponds to the sixth embodiment, the example is also applicable to other embodiments.

In the above-described individual embodiments, a solution-temperature monitoring means that monitors the solution temperature may be installed in the solution-holding means. In this case, the solution-temperature monitoring means may be configured so as to transmit the monitored solution-temperature information to the remote control portion. By doing so, it is possible to prevent the solution at a temperature that is not appropriate for cell culturing from being accidentally supplied.

In the above-described individual embodiments, although forms in which two solution-holding means (and solution-stocking means) are installed have been described, three or more solution-holding means (and solution-stocking means) may be installed. In this case, it is possible to use multiple types of mediums having different compositions. In addition, a form in which one solution-holding means (and solution-stocking means) is provided and a solution-holding means (and solution-stocking means) for cleaning liquid is not used may be employed.

In the above-described individual embodiments, the control portion 19 may be configured so as to allow information exchange with the temperature-controlling means 2, the pressurizing means 60, and the negative-pressure supplying means 71 of the above-described individual embodiments via wireless or wired communication and so as to allow remote control thereof. By doing so, it is possible to increase the efficiency of work remotely performed by the user.

In the above-described individual embodiments, although one (or two) cell-culturing container has been described, in reality, it is possible to install a plurality of cell-culturing containers. In the case in which a plurality of cell-culturing containers are installed, the descriptions of the above-described individual embodiments and modifications are applicable to the individual cell-culturing containers thereof.

In the above-described individual embodiments, although the forms in which the waste liquid is sucked out by using the negative-pressure supplying means has been described, as shown in Fig. 36, the waste liquid may be discharged by the action of gravity without employing the negative-pressure supplying means. In this case, the discharging port of the cell-culturing container may be disposed at a bottom surface or a side surface of the cell-culturing container, and the solution in the cell-culturing container may be discharged from the discharging port by the action of gravity. In addition, in order to discharge nearly the entire amount of solution in the cell-culturing container, it is preferable that the cell-culturing container be installed in an inclined manner so that the solution flows toward the discharging port.

In addition, as shown in Fig. 37, a liquid-feeding pump 53, such as a peristaltic pump or the like, may be installed in a tubular member leading to the discharging port of the cell-culturing container. In this case, it is possible to employ a form in which the position at which the discharging port of the cell-culturing container is installed is set in the lid at the top surface of the cell-culturing container, and the tubular member extends therefrom into the interior of the cell-culturing container.

In the above-described individual embodiments, although forms in which the user remotely performs the medium replacement by means of the control portion have been described, the control portion may remotely perform the medium replacement on the basis of a schedule (program) set by the user in advance.

In the above-described individual embodiments, although forms in which the solution-holding means is installed inside the incubator have been described, the solution-holding means may be installed outside the incubator. In this case, it is preferable that the temperature-controlling means that maintains the solution in the solution-holding means at an appropriate temperature for cell culturing (for example, 37 °C) be installed.

Examples of the control portions of the present invention include a PC, and the PC can perform control executed by the control portions 19 in the above-described individual embodiments.

In the forms in which two (plurality of) supply ports of the cell-culturing container are provided in the above-described individual embodiments, a single supply port may be provided and tubular members leading thereto from the two (plurality of) solution-supplying means may be connected to that single supply port after being joined together.

A sac-like cell-culturing bag may be employed as the cell-culturing container to be used in the present invention.

Examples of the culturing container that can be used in the present invention include a container shown in Figs. 39A and 39B in which a lid portion 151 is made of a material that can be subjected to heat sterilization. This lid portion 151 has a structure in which at least one hole 154 is provided in a lid main body 152 and a tubular member 153 passes therethrough. Although Figs. 39A and 39B show a case in which two each of the holes 154 and the tubular members 153 are provided, three or more of each or one of each may be provided. The lid portion 151 is made of a material that can resist intense heat when subjected to sterilization by means of a dry-heat treatment or an autoclave. Examples of materials that can resist intense heat include metals, such as aluminum or the like, and glass. A container main-body portion 155 need not be made of a material that can resist intense heat. By doing so, it is possible to dispose the container main body 155 after using the culturing container, and to reuse the lid portion by combining with a new container main body after being washed and sterilized. It is preferable that the tubular member 153 be movable in the insertion direction via the hole 154. By doing so, it is possible to adjust the length by which the tubular member 153 protrude into the interior of the container, and thus, it is possible to perform appropriate adjustment to achieve optimum conditions depending on the types of containers and culturing system to be used.

With this culturing container, it is possible to reuse the lid portion, which makes it advantageous in terms of cost. In addition, because the container main-body portions are replaceable, it is possible to appropriately select an optimum container depending on the culturing system, which increases the variation of the culturing system, and which is also advantageous in terms of costs when commercially available container main-body portions are used.

With the present invention, it is possible to provide a cell-culturing system including: a solution-holding means that holds a solution, such as a medium or the like for culturing cells; a solution-supplying means that is connected to the solution-holding means and that supplies the solution supplied thereto from the solution-holding means to a cell-culturing container; and a solution-discharging means that discharges the solution from the cell-culturing container, the cell-culturing system being characterized in that the solution-supplying means supplies the solution to the cell-culturing container via a solution dripping step.

With the present invention, it is possible to provide a cell-culturing system including: a solution-holding means that holds a solution, such as a medium or the like for culturing cells; a solution-supplying means that is connected to the solution-holding means and that supplies the solution supplied thereto from the solution-holding means to a cell-culturing container; and a solution-discharging means that discharges the solution from the cell-culturing container, the cell-culturing system being characterized in that the solution-discharging means discharges the solution via a solution dripping step.

### {Reference Signs List}

- 1: incubator
- 2: temperature-controlling means
- 3: medium-preserving means (solution-holding means)
- 4: temporary holding means
- 6: medium-supplying means
- 7: dripping-speed adjusting means
- 8: dripping container
- 11: cell-culturing container
- 14: waste-liquid holding means
- 17: waste-liquid-speed adjusting means
- 19: control portion
- 20: tubular member (tube)
- 21: plate-like member
- 22: spherical (columnar) member
- 24: shutter-like member
- 23, 25, 26: through-hole
- 31: pump
- 32: waste-liquid container
- 33: suction port
- 41: dripping container
- 51: solution-stocking means
- 52: temperature-controlling means
- 53: pump
- 60: pressurizing means
- 61: sac-like member
- 62, 64: isolating member
- 65: weight
- 71: negative-pressure supplying means
- 81: pump
- 82: waste-liquid container
- 83: suction port
- 91: culturing container
- 92: lid portion
- 95: supplying-pipe portion
- 96: discharging-pipe portion
- 97: neck portion
- 101: protrusion
- 102: ring-like member
- 103: depression
- 111: discoid member
- 112: lid main body
- 113: ring-like member
- 121: liquid-feeding pump
- 130: plate-like member
- 131: solution stopper
- 132: opening
- 133: supply port
- 134: discharging port
- 151: lid portion
- 152: lid main body
- 153: tubular member
- 154: hole
- 155: container main body

## Claims

1. A cell-culturing system comprising:
a solution-holding means that holds a solution, such as medium or the like for culturing cells; and
a solution-supplying means that is connected to the solution-holding means and that supplies the solution supplied thereto from the solution-holding means to a cell-culturing container
wherein the solution-supplying means supplies the solution to the cell-culturing container via a solution dripping step.

2. A cell-culturing system according to Claim 1,
wherein the cell-culturing container is connected to the solution-supplying means at a position that is lower than the solution-supplying means in a direction in which gravity acts,
the solution-supplying means has a space in which the solution supplied thereto from the solution-holding means is made to drip,
the cell-culturing container is provided with a discharging port that discharges the solution at a position in a container side surface at a desired height, and
the solution is discharged outside the cell-culturing container from the discharging port when the height of a top surface of the solution in the cell-culturing container reaches the discharging port as a result of supplying the solution to the cell-culturing container from the solution-supplying means.

3. A cell-culturing system according to Claim 1,
wherein the cell-culturing container is provided with a supply port to which the solution is supplied from the solution-supplying means at a container top surface, and is also provided with a discharging port that discharges the solution at a position in a container side surface at a desired height,
the solution supplied from the supply port is made to drip in a space inside the cell-culturing container toward a top surface of the solution, and
the solution is discharged outside the cell-culturing container from the discharging port when the height of the top surface of the solution in the cell-culturing container reaches the discharging port as a result of supplying the solution to the cell-culturing container from the solution-supplying means.

4. A cell-culturing system according to Claim 1,
wherein the solution-supplying means is provided with:
a dripping container having a space in which the solution supplied thereto from the solution-holding means is made to drip; and
a dripping-speed adjusting means that controls a dripping speed of the solution in the dripping container.

5. A cell-culturing system according to Claim 1, further comprising:
a dripping-speed adjusting means that controls a dripping speed of the solution in the cell-culturing container.

6. A cell-culturing system according to Claim 4 or 5, further comprising:
a control portion that remotely controls the dripping-speed adjusting means.

7. A cell-culturing system according to any one of Claims 1 to 6, further comprising:
a pressurizing means that applies pressure to the solution in the solution-holding means.

8. A cell-culturing system according to any one of Claims 1 to 7, further comprising:
a negative-pressure supplying means that applies negative pressure to a discharging port of the cell-culturing container.

9. A cell-culturing system according to any one of Claims 1 to 8, further comprising:
a waste-liquid holding means that is connected to a discharging port of the cell-culturing container and that holds the medium discharged from the cell-culturing container,
wherein the medium discharged from the cell-culturing container is made to drip in a space in the waste-liquid holding means.

10. A cell-culturing system according to Claim 1, further comprising:
a solution-discharging means that discharges the solution from the cell-culturing container,
wherein the cell-culturing container is connected to the solution-supplying means at a position that is lower than the solution-supplying means in a direction in which gravity acts, and
the solution-supplying means has a space in which the solution supplied thereto from the solution-holding means is made to drip.

11. A cell-culturing system according to Claim 1, further comprising:
a solution-discharging means that discharges the solution from the cell-culturing container,
wherein the cell-culturing container is provided with, at a container top surface, a supply port to which the solution is supplied from the solution-supplying means, and
the solution supplied to the supply port from the solution-supplying means is made to drip in a space in the cell-culturing container from the supply port.

12. A cell-culturing system according to Claim 10 or 11,
wherein the solution-supplying means is provided with a dripping-speed adjusting means that controls a dripping speed of the solution,
the solution-discharging means is provided with a waste-liquid-speed adjusting means that controls a discharging speed of the solution, and
the cell-culturing system further comprising:
a control portion that remotely performs the control of the dripping speed of the solution by means of the dripping-speed adjusting means and the control of discharging speed of the solution by means of the waste-liquid-speed adjusting means.

13. A cell-culturing system according to any one of Claims 10 to 12, further comprising:
a pressurizing means that applies pressure to the solution in the solution-holding means.

14. A cell-culturing system according to any one of Claims 10 to 13, wherein the cell-culturing container is provided with a discharging port that is connected to the solution-discharging means,
the cell-culturing system further comprising:
a negative-pressure supplying means that applies negative pressure to the discharging port of the cell-culturing container.

15. A cell-culturing system according to any one of Claims 10 to 14,
wherein the solution-discharging means is provided with a waste-liquid holding means that is connected to the cell-culturing container and holds the solution discharged from the cell-culturing container, and
the solution discharged from the cell-culturing container is made to drip in a space in the waste-liquid holding means.

16. A cell-culturing system comprising:
a solution-holding means that holds a solution, such as a medium or the like for culturing cells;
a solution-supplying means that is connected to the solution-holding means and that supplies the solution supplied thereto from the solution-holding means to a cell-culturing container; and
a solution-discharging means that discharges the solution from the cell-culturing container,
wherein the solution-discharging means discharges the solution from the cell-culturing container via a solution dripping step.
